# EUROPÄISCHE PATENTSCHRIFT

(11) **EP 1 624 904 B1**
(45) Veröffentlichungstag und Bekanntmachung des Hinweises auf die Patenterteilung: **03.01.2007**
(21) Anmeldenummer: 04739387.1
(22) Anmeldetag: 24.05.2004
(51) Int. Cl.: A61L 27/12, A61L 27/42, A61L 27/56, A61L 27/58

(54) **ANORGANISCHES RESORBIERBARES KNOCHENERSATZMATERIAL**
INORGANIC RESORBABLE BONE SUBSTITUTE MATERIAL
MATERIAU DE REMPLACEMENT OSSEUX INORGANIQUE RESORBABLE

(30) Priorität: 22.05.2003 DE 10323079; 22.08.2003 DE 10338634
(43) Veröffentlichungstag der Anmeldung: 15.02.2006
(73) Patentinhaber: Artoss GmbH, 18119 Rostock (DE)
(72) Erfinder: GERBER, Thomas, 18059 Sildemow (DE)
(74) Vertreter: Weber-Quitzau, Martin
(86) Internationale Anmeldenummer: PCT/EP2004/005709
(87) Internationale Veröffentlichungsnummer: WO 2004/103421

(56) Entgegenhaltungen:
- EP-A- 1 281 383
- WO-A-02/085250
- WO-A-03/093196
- DE-A- 19 825 419
- DE-C- 10 060 036
- US-A- 5 650 108
- US-A- 5 997 624

## Beschreibung

Die Erfindung betrifft insbesondere ein Hydroxylapatit/Siliziumdioxid Granulat definierter Morphologie, ein auf diesem Granulat basierenden hochporösen Knochenersatzmaterial und einer wiederum darauf basierenden Glaskeramik als Knochenersatzmaterial, die sich durch eine variable mechanische Festigkeit auszeichnet, und Formkörper' aus diesem Material, wobei im Formkörper bevorzugt Materialien unterschiedlicher mechanischer Festigkeit verwendet werden. Die erfindungsgemäßen Knochenersatzmaterialien zeichnen sich durch eine gute Resorbierbarkeit in vivo aus.

Die Transplantation von Knochen ist nach der Applikation von Blutbestandtteilen die zweithäufigste Transplantationsform beim Menschen (Fox, R.: New bone The Lancet 339, 463f. (1992)). So wurden in den USA 1993 250000 Knochentransplantationen durchgeführt (Kenley et al.: Biotechnology and bone graft substitutes. Pharmaceut. Res. 10, 1393 (1993)). Der Ersatz kongenitaler, posttraumatischer, als Folge von Osteomyelitiden und Tumoroperationen auftretender sowie osteoporotischer Knochendefekte ist von eminenter klinischer Bedeutung, da nur auf diese Weise eine funktionell umfassende Rehabilitation möglich ist.

In der Literatur werden eine Vielzahl von porösen Materialien als Knochenersatz beschrieben. 1992 wurde eine aus Rinderknochen hergestellte Keramik publiziert, wobei die gesamte organische Matrix entfernt und der keramische Anteil bei Temperaturen von 1100°C bis 1500°C getempert wird (Bauer G, Vizethum, F. Process for producing a bone replacement material. US Patent 5,133,756; 1992).

Einige Verfahren zur Herstellung von porösen Knochenersatzstoffen nutzen das Gerüst natürlicher Korallen (Pollick S, Shors, EC, Holmes RE, Kraut RA. Bone formation and implant degradation of coralline porous ceramics placed in bone and ectopic sites. J Oral Maxillofac Surg 1995; 53(8): 915-23, White, EW. Calcium phosphate bone substitute materials. US Patent 4,861,733; 1989), die eine ideale Porenstruktur (Größenverteilung, Morphologie) für das Einwachsen des Knochengewebes aufweisen.

Der entscheidende Nachteil dieser Keramiken ist, daß sie nicht resorbierbar sind (Jenssen SS, Aaboe M, Pinholt EM, Hjorting-Hansen E, Melsen F, Ruyter IE. Tissue reaction and material characteristics of four bone substitutes. Int J Oral Maxillofac Implants. 1996; 11(1): 55-66). Gebildeter Knochen unterliegt einem ständigen Umbau, auch als Remodeling bezeichnet, wobei Osteoklasten den Knochen abbauen und die Osteoblasten ihn wieder aufbauen. Für die beschriebenen Materialien bedeutet das, dass das Knochengewebe zwar ausgezeichnet in die Porenstruktur hineinwächst, während der hochkristalline Hydroxylapatit der Keramik allerdings nicht am Knochenremodeling beteiligt ist. Er bleibt daher ein Fremdkörper und beeinflusst die mechanischen Eigenschaften des Knochenregenerates ungünstig. Zudem kommt es zu einer Entzündungsreaktion im Grenzbereich von Gewebe und Keramik (Günther KP, Scharf H-P, Pesch H-J, Puhl W. Einwachsverhalten von Knochenersatzstoffen. Orthopädie 1998; 27: 105-117, Sailer JD, Weber FE. Knochenersatzmaterialien. Mund Kiefer Gesichts Chir 2000; 4 (Suppl.1) 384-391).

Poröse, auf Hydroxylapatit (HA) basierende Materialien sind ein idealer Knochenersatz, da sie durch eine spezielle Oberflächencharakteristik die Geweberegeneration fördern. In der Literatur wird im allgemeinen festgestellt, dass diese Keramiken jedoch nicht im eigentlichen Sinne osteoinduktiv wirken (Heymann D, Delecrin J, Deschamps C, Gouin F Padrines M, Passuti N. In vitro assessment of associating osteogenic cells with macroporous calcium-phosphate ceramics. Rev Chir Orthop Reparatrice Appar Mot 2001; 87(1): 8-17, Osborne JF, Newesely H. The material science of calcium phosphate ceramics. Biomaterials 1980; 1: 108-112, Vuola J, Taurio R, Goransson H, Asko-Seljavaara S. Compressive strength of calcium carbonate and hydroxyapatite implants after bone-marrow-induced osteogenesis. Biomaterials 1998; 19(1-3): 223-7). Es erfolgt vielmehr ein stoffschlüssiger Knochenanbau (bonding) durch die Proteinadsoption und die Anlagerung von Osteoblasten an eine primär das Implantat bedeckende biologische Apatitschicht (De Bruijn JD, Klein CPAT, De Groot K, Van Blitterswijk CA. Ultrastructure of the bonehydroxylapatit interface in vitro. J Biomed Mater Res. 1992; 26: 1365-1382, Donath K, Hormann, K, Kirsch A. Welchen Einfluss hat Hydroxylapatitkeramik auf die Knochenbildung? Dtsch Z Mund Kiefer Gesichtschir. 1985; 9(6): 438-40).

Dagegen stellt Yuan et. al (Yuan H, Kurashina K, de Bruijn JD, Li Y, de Groot K, Zhang X. A preliminary study on osteoinduction of two kinds of calcium phosphate ceramics. Biomaterials 1999; 20(19): 1799-806) fest, dass in Abhängigkeit von der Mikrostruktur der Keramik bei gleicher chemischer und kristallographischer Struktur des Kalziumphosphates osteoinduktive Eigenschaften hervorgerufen werden können.

Das bedeutet, dass diese Materialien eine dystope Knochenbildung induzieren können, z.B. wenn sie unter die Haut oder ins Muskelgewebe implantiert werden, wo keine anderen osteoinduktiven Stimuli vorhanden sind. Diese osteoinduktive Eigenschaften (bone formation in extraosseal sites) wird bei verschiedenen Hydroxylapatit-Keramiken (HA-Keramiken) ebenfalls hervorgerufen, wenn sie mit Knochenmarkzellen getränkt wurden (Heymann D, Delecrin J, Deschamps C, Gouin F Padrines M, Passuti N. In vitro assessment of associating osteogenic cells with macroporous calcium-phosphate ceramics. Rev Chir Orthop Reparatrice Appar Mot 2001; 87 (1) : 8-17, Vuola J, Taurio R, Goransson H, Asko-Seljavaara S. Compressive strength of calcium carbonate and hydroxyapatite implants after bone-marrow-induced osteogenesis. Biomaterials 1998; 19(1-3): 223-7) .

Dagulsi beschreibt die Zellreaktion, Biodegradation und - resorption sowie die Transformation in Karbonat-Hydroxylapatit eines zweiphasigen Materials (HA/TCP), das als Formkörper, Beschichtung als auch injizierbares Knochenersatzmaterial verwendet wurde (Dagulsi G. Biphasic calcium phosphate concept applied to artificial bone, implant coating and injectable bone substitute. 1998, 19(16): 1473-8).

Im Rahmen der Entwicklung eines resorbierbaren Knochenersatzstoffes wurde der Einfluss verschiedener Kalziumphosphate und Kombinationen von Kalziumphosphaten auf die Entwicklung von Osteoblasten in vitro untersucht. In einer vergleichenden Studie implantierten Oonishi et al. verschiedene biokeramische Materialien in Femurkondylen ausgewachsener Japanischer Weißer Kaninchen und geben im Ergebnis folgende Resorptionsaktivitäten an: HA mit geringem Kristallinitätsgrad, OCP > TeCP, TeDCPD, TeDCPA > αTCP, βTCP (Oonishi H, Hench LL, Wilson J, Sugihara F, Tsuji E, Kushitani S, Iwaki H. Comparative bone growth behavior in granules of bioceramic materials of various sizes. J Biomed Mater Res 1999; 44 (1) : 31-43).

Sun et al. stellten dabei fest, dass eine Kombination von Hydroxylapatit und β-Trikalziumphosphat (βTCP) eine hemmende Wirkung auf das Wachstum der Osteoblasten hat (Sun JS, Tsuang YH, Liao CJ, Liu, HC, Hang YS, Lin FH. The effects of calcium phosphate particles on the growth of osteoblasts. J Biomed Mater Res 1997; 37(3): 324-334).

Auch der Einfluss verschiedener resorbierbarer Keramiken, wie z.B. CaNaPO₄, CaNaPO₄+MgNaPO₄, CaNaPO₄+Mg₂SiO₄ u.a., auf das Wachstum der Osteoblasten wurde in vitro untersucht (Knabe C, Gildenhaar R, Berger G, Ostapowicz W, Fitzner R, Radlanski RJ, Gross U. Morphological evaluation of osteoblasts cultured on different calcium phosphate ceramics. Biomaterials 1997; 18(20): 1339-1347). Die beste Unterstützung des Wachstums der Osteoblasten wurde bei CaNaPO₄+MgNaPO₄ und bei Ca₂KNa (PO₄)₂ gefunden. Werden von der Keramik zu viele Ca²⁺-Ionen abgegeben, wird das Zellwachstum gehemmt.

Oonishi et al. vergleichen in einer Studie an Femurkondylen ausgewachsener Kaninchen das Einwachsverhalten von Granulaten eines Bioglases und synthetischem, temperaturbehandeltem Hydroxylapatit (Oonishi H, Hench LL, Wilson J, Sugihara F, Tsuji E, Matsuura M, Kin S, Yamamoto T, Mizokawa S. Quantitative comparison of bone growth behavior in granules of Bioglass, A-W glass-ceramic, and hydroxyapatite. J Biomed Mater Res 2000; 51(1): 37-46). Im Gegensatz zum Bioglas wird der synthetische Hydoxylapatit nicht vollständig resorbiert.

Bioaktive Gläser werden ebenfalls als Knochenersatzmaterial beschrieben (US 6,054,400; 2000; US 5,658,332; 1997). Das anorganische Material liegt hier als glasiger Festkörper vor. Poren in der Größenordnung der Spongiosa erlauben ein Einwachsen des Gewebes. Kleinere Poren liegen in dem Material nicht vor.

Auch Glaskeramiken werden als Knochenersatz angeboten (z.B. US 5,981,412; 1999). Sie sind mit den bioaktiven Gläsern zu vergleichen, wobei in die Glasmatrix, die im Allgemeinen ein bioaktives Calciumsilicatglas ist, kristalline Komponenten wie z.B. Na₂O 2CaO·3SiO₂ eingelagert sind.

Als weitere Stoffgruppe für den Einsatz als Knochenersatz wurden Calciumphosphatzemente entwickelt (US 5,997,624; 1999; US 5,525,148; 1996). Ein entscheidender Nachteil dieser Stoffgruppe ist, dass keine definierten interkonnektierenden Poren in das Material eingebracht werden, womit sie auf sehr kleine Knochendefekte beschränkt sind.

In den Patenten DE 198 25 419 und DE 100 03 824 wurden Verfahren dargestellt, mit denen hochporöse Calciumphosphatkeramiken auf der Basis von Hydroxylapatit unter Verwendung der Sol-Gel-Technik hergestellt werden können, die speziell für die Auffüllung und Rekonstruktion von Knochendefekten verschiedener Größe bestimmt ist. Die Verfahren zielen darauf ab, hochporöse Strukturen zu erzeugen. Mit dem Verfahren des Patentes DE 198 25 419 wird eine Porosität von bis zu 70% erreicht, wobei die Poren im Bereich von 1-10 Mikrometer liegen. Mit dem Patent DE 100 03 824 wird ein Verfahren beschrieben, das zusätzlich eine Porenstruktur im Größenbereich von 0,1 bis ca. 1 Millimeter erzeugt, wie sie auch in der natürlichen Spongiosa vorliegt.

In DE 100 60 036 wird ein anorganisches resorbierbares Knochenersatzmaterial beschrieben, das ein lockeres Kristallgefüge besitzt, d.h. die Kristallite sind nicht wie in einem Festkörper (Keramik) dicht zusammengefügt, sondern nur über einige Molekülgruppen miteinander verbunden. Das Volumen, das im natürlichen Knochen vom Kollagen eingenommen wird, ist in dem Material als interkonnektierende Poren im Nanometerbereich vorhanden. Eine zweite Porengröße, ebenfalls interkonnektierend und im Bereich von einigen Mikrometern, ermöglicht ein Einwachsen von Kollagenfasern bei der Gewebebildung. Diese Fasern sind Keimbildner für die einsetzende Biomineralisierung (Bildung des körpereigenen biologischen Apatits). Das Material enthält eine dritte interkonnektierende Porenkategorie, die der Spongiosa nachempfunden ist, im Bereich von ca. 100µm bis 1000µm liegt und damit ein Einwachsen von Blutgefäßen ermöglicht, wodurch die Resorption und die Knochenneubildung nicht nur als Front vom gesunden Knochen aus erfolgt, sondern aus dem gesamten Defekt heraus geschieht.

Bei diesem Material steht die Förderung der Osteogenese und die Resorptionseigenschaft im Vordergrund, so dass das Remodellierung des Knochens unterstützt wird.

In der einschlägigen Fachliteratur wird darauf verwiesen, dass Knochenersatzmaterialien auf der Basis von Hydroxylapatit praktisch nicht resorbiert werden und einen Fremdkörper auf Dauer darstellen. Im Gegensatz dazu wird das in DE 100 60 036 beschriebene Material, das im wesentlichen aus Hydroxylapatit besteht, sehr gut resorbiert und gleichzeitig die Knochengewebsneubildung beschleunigt. Diese Eigenschaft wird durch das beschriebene lockere Kristallgefüge von Kalziumphosphaten determiniert.

Die mechanische Festigkeit dieses Materials ist jedoch relativ gering. Es kann keine mechanische Stützfunktion übernehmen. Darüber hinaus sind die Variationsmöglichkeiten des Knochenersatzmaterials, um für den Ersatz ganzer Knochenfragmente (z.B. Teile eines Röhrenknochens) verwendet werden zu können, sehr beschränkt.

In der Rekonstruktionschirurgie und in der Orthopädie werden insbesondere bei größeren Defekten Knochenersatzmaterialien benötigt, die Komponenten mit höherer mechanischer Festigkeit enthalten. Im Zusammenhang mit Computertomographie am Patienten und computergestützter Fertigung können z.B. Ersatzpartien der Schädelknochens nachgeformt werden.

Der vorliegenden Erfindung liegt demgegenüber die Aufgabe zugrunde, einen Knochenersatzwerkstoff bereitzustellen, der eine Bildung von Knochengewebe unterstützt (der also osteokonduktiv bzw. osteoinduktiv ist), der über die natürlichen Prozesse des Knochenremodeling resorbiert wird und der eine mechanische Festigkeit besitzt, die den verschiedenen Anwendungen entsprechend angepasst werden kann. Defekte im Knochen, die z.B. durch Entzündungen entstehen, sind meist von mehreren Seiten von gesundem Knochen umgeben. Für diese Defekte spielt die mechanische Festigkeit des Knochenersatzmaterials keine Rolle. Fehlen jedoch durch einen Trümmerbruch oder durch das Entfernen eines Knochentumors ganze Knochensegmente, muss das Knochenersatzmaterial eine tragende Funktion übernehmen. In diesem Fall wird aus dem Knochenersatzmaterial ein Ersatzknochen (z.B. für ein fehlendes Stück Röhrenknochen ein Hohlzylinder )aus dem Knochenersatzmaterial gefertigt, das dann mit Osteosyntheseplatten (Metallplatten, die nach der Heilung wieder entfernt werden) mit dem verbliebenen Knochen verschraubt wird. Die tragende Funktion übernimmt jetzt das System aus Ersatzknochen aus Knochenersatzmaterial und Osteosyntheseplatte. Da sicher ist, das eine erhöhte mechanische Festigkeit eine verschlechterte Resorption zur Folge hat, muss je nach Defektgröße und mechanischer Belastung ein Kompromiss in den Materialeigenschaften eingegangen werden.

Zur Lösung der Aufgabe werden Granulate, auf diesen Granulaten basierende hochporöse Knochenersatzmaterialien, darauf basierende Glaskeramiken als Knochenersatzmaterialien mit variabler mechanischer Festigkeit, Verwendungen, Mittel, Formkörper, Verfahren etc. vorgeschlagen. Zur Lösung werden insbesondere die Produkte der angefügten Ansprüche 1 bis 27, 34 und 61, die Verfahren der angefügten Ansprüche 35 bis 57 und die Verwendungen der angefügten Ansprüche 28 bis 33 und 58 bis 60 vorgeschlagen.

Die Aufgabe wird somit erfindungsgemäß durch ein Material gelöst, das kristallines Calciumphosphat in eine Xerogelmatrix eingebettet enthält. Diese Xerogelmatrix besteht aus Siliziumdioxid.

Ein Xerogel ist ein trockenes Gel, das durch eine hohe innere Oberfläche und eine unvollständige Vernetzung der Baugruppen charakterisiert ist.

Damit liegt ein vollkommen neuer Materialtyp vor, der vergleichbar mit einer Glaskeramik ist, wobei nun die Matrix, die die kristallinen Komponenten enthält, kein Glas sondern ein Xerogel mit seiner typischen porösen Struktur ist. Die Xerogelmatrix soll vorzugsweise einen Gewichtsanteil von 4 bis 80% bezogen auf die Gesamtmasse des Knochenersatzmaterials einnehmen. Da ein Silicaxerogel ein poröses Material ist, in dem SiO₄/2 Tetraeder locker verknüpft sind und das eine hohe innere Oberfläche mit -SiOH Gruppen besitzt, kann in Abhängigkeit von der Größe der Kristallite des Calciumphosphates schon mit geringen Gewichtsanteilen eine Matrix aufgebaut werden, die die kristallinen Komponenten umschließt. In Abhängigkeit von der Größe der Kristallite ist eine Reduktion des Matrixanteils bis unter 5 Gew.-% möglich.

Die Xerogelmatrix hat verschiedene Aufgaben. Zum einen verbindet sie natürlich die kristallinen Komponenten des Materials. Durch die relativ lockere Verknüpfung des Siliziumdioxids ist die mechanische Festigkeit des Materials eingeschränkt. Die Bruchfestigkeit liegt typischerweise im Bereich von 2 bis 15 MPa (siehe Beispiel 6). Zum anderen erlaubt die Porosität des Xerogels die Resorption des Biomaterials und verbessert die Bioaktivität, die natürlich in erster Linie durch die Calciumphosphatkomponenten gegeben ist, indem sich während der Anwendung an die hohe innere Oberfläche körpereigene Proteine aus dem Blut des Patienten anlagern. Die Zellen stufen daher das Biomaterial nicht als körperfremd ein.

Gegenstand der Erfindung ist somit ein Granulat und eine darauf basierende Gruppe von Knochenersatzmaterialien, die im weiteren beschrieben werden. Das Granulat basiert auf Calciumphosphat, bei dem kristallines Calciumphosphat in einer Siliziumdioxid-Xerogelmatrix eingebettet ist, wobei die Kristallite einen mittleren Durchmesser von 10 nm bis 2000 nm, bevorzugt von 10 nm bis 200 nm aufweisen, wobei besonders bevorzugt plättchenförmige Kristallite mit einer Dicke von 2.5 nmm bis 10 nm und einem mittleren Durchmesser von 10 nm bis 200 nm enthalten sind. Die Granulatkörner weisen einen mittleren Durchmesser von etwa 1 µm bis etwa 1000 µm auf und der Siliziumdioxid-Anteil im Bereich von etwa 2 bis etwa 80 Gew.-%, vorzugsweise im Bereich von etwa 4 bis etwa 50 Gew.-% liegt.

Die Poren im Xerogel weisen einen durchschnittlichen Durchmesser im Bereich von 0,5 nm bis 20 nm auf. Sie machen in den Granulatkörnern jeweils etwa 10 Vol.-% bis etwa 60 Vol.-% bezogen auf das Volumen des Granulatkorns aus.

Vorzugsweise ist das Calciumphosphat Hydroxylapatit.

Das Granulat kann in einer besonderen Ausführungsform ferner lösliches Calciumphosphat umfassen, wobei das lösliche Calciumphosphat vorzugsweise in einem Anteil von etwa 5 Gew.%. bis 50 Gew.-% bezogen auf den Calciumphosphatanteil vorliegt. Das lösliche Calciumphosphat ist insbesondere β-Tricalciumphosphat (βTCP).

Das Xerogel des Granulats kann ferner ein oder mehrere Netzwerkwandleroxide umfassen. Das (die) Netzwerkwandleroxid(e) liegt (liegen) bevorzugt in einem Anteil von 0,5 bis 35 Mol.-%, vorzugsweise in einem Anteil von 17 Mol.% bis 30 Mol.-%, bezogen auf das Siliziumdioxid vor. Das Netzwerkwandleroxid ist insbesondere Na₂O.

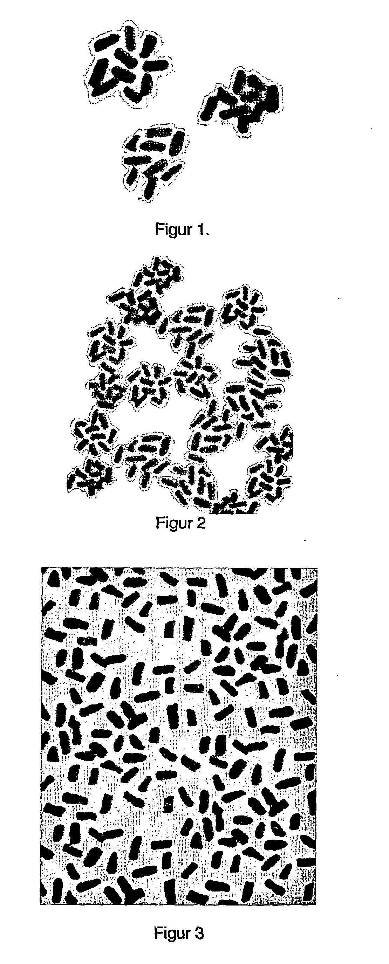

In Fig. 1 ist ein erfindungsgemäßes Granulatteilchen beispielhaft schematisch dargestellt. Die Kristallite (schwarz dargestellt) im Granulat werden durch das SiO₂-Xerogel (grau dargestellt) zusammengehalten. An der Oberfläche der Granulatteilchen befindet sich SiO₂-Xerogel. Es soll kurz bemerkt werden, das ein Granulatkorn aus dem bevorzugten Größenbereich mit einem Durchmesser von z.B. 1 µm in der Größenordnung 10⁴ Kristallite enthält, wenn diese z.B. Plättchen mit einem Durchmesser von 100 nm und einer Dicke von 10 nm sind und die Xerogelmatrix 40 Gew.-% des Granulatkorns einnimmt.

Aufbauend auf dem beschriebenen Hydroxylapatit/Siliziumdioxid Granulat erhält man ein hochporöses Knochenersatzmaterial und eine Glaskeramik als Knochenersatzmaterial mit variabler mechanischer Festigkeit.

Ausgangspunkt ist ein hochporöses Knochenersatzmaterial, das dadurch gekennzeichnet ist, das die Granulatkörner miteinander über die Xerogelmatrix verbunden sind und durch die Packung der Granulatkörner Poren entstehen, die in der Größenordnung der Granulatkörner liegen. Das hochporöse Knochenersatzmaterial hat demnach zwei Porenkategorien. Neben den eben beschriebenen Poren, die durch die Packung der Granulatkörner zustande kommen und damit im Mikrometerbereich liegen, sind die Poren, die innerhalb des Granulates liegen und die oben beschrieben wurden, weiterhin vorhanden. Es sind die Poren im Xerogel, die einen durchschnittlichen Durchmesser im Bereich von 0.5 nm bis 20 nm aufweisen.

Damit liegt in dem hochporösen Knochenersatzmaterial eine Porosität von bevorzugt 30 Vol.-% bis 80 Vol.-% vor.

In Fig. 2 ist die Struktur des hochporösen Knochenersatzmaterials schematisch dargestellt. Ein wesentlicher Unterschied zu Knochenersatzmaterialien im Stand der Technik besteht darin, dass das Innere der Granulatteilchen (d.h. die Kristallite) definiert durch SiO₂ zusammengehalten wird. Die Struktur kann so beschrieben werden, dass jeder einzelne Kristallit in einer Xerogelmatrix liegt. Das Produkt ist durch z.T. herkömmliche Keramikherstellungsprozesse bei Verwendung der beschriebenen Granulate erhältlich, wie weiter unten ausführlich beschrieben werden.

Die Erfindung betrifft ferner ein hochporöses Knochenersatzmaterial, das Granulatkörner des vorgenannten Granulats umfasst, die eine dreidimensionale Struktur bilden, die neben den in den Granulatkörnern vorhandenen Poren ferner Poren in etwa der Größe der Granulatkörner aufweist. Die Porendurchmesser liegen damit im Bereich von 1 µm bis 1000 µm, bevorzugt im Bereich von 1 µm bis 50 µm.

Kleine Stücke (z.B. Formkörper, Teilchen, Körper) aus diesem hochporösen Knochenersatzmaterial, bevorzugt in der Form von Zylindern mit einem mittleren Durchmesser von etwa 0,4 bis etwa 2 mm und einer Länge von 1 bis 6 mm, dienen zur Auffüllung von kleinen Knochendefekten, bevorzugt bis zu einer Größe von 10 cm³ insbesondere, wenn die Defekte bis auf zwei Seiten von gesundem Knochen begrenzt sind.

Die Erfindung betrifft somit ferner ein hochporöses Knochenersatzmaterial, das dadurch gekennzeichnet ist, dass es weiterhin (d.h., zusätzlich zu den Poren innerhalb der einzelnen Granulatkörner und zusätzlich zu den Poren, die durch die (dreidimensionale) Granulatkörnerpackung zustande kommt) interkonnektierende Makroporen im Bereich von etwa 100 µm bis zu mehreren 1000 µm aufweist, die einen Volumenanteil von 10 Vol.-% bis 60 Vol.-% haben. Damit hat das hochporöse Knochenersatzmaterial bevorzugt eine Gesamtporosität von 30 Vol.-% bis 90 Vol.-%, besonders bevorzugt eine Gesamtporosität von 60 Vol.-% bis 80 Vol.-%.

Die Bruchfestigkeit des hochporösen Knochenersatzmaterials ohne die beschrieben Makroporen, liegt bei 2 MPa bis zu 15 MPa, vorzugsweise 3 bis 10 MPa. Durch die Makroporen sinkt die Bruchfestigkeit des Materials und erreicht nur noch Werte von 0,1 MPa bis 4 MPa.

Gemäß einer besonderen Ausführungsform umfasst das hochporöse Knochenersatzmaterial ferner ein oder mehrere Netzwerkwandleroxide. Das (die) Netzwerkwandleroxid(e) liegen bevorzugt in einem Anteil von 0,5 bis 35 Mol.-%, vorzugsweise in einem Anteil von 17 bis 30 Mol.-%, bezogen auf das Siliziumdioxid vor. Na₂O ist besonders bevorzugt.

Die Erfindung betrifft ferner eine Glaskeramik als Knochenersatzmaterial (oder - anders ausgedrückt, ein eine Glasmatrix umfassendes Knochenersatzmaterial), die (das) dadurch gekennzeichnet ist, dass kristallines Calciumphosphat in eine Glasmatrix eingebettet ist, wobei die Kristallite eine Größe von 10 nm bis 2000 nm aufweisen und der Glasanteil im Bereich von 4 bis 80 Gew.-% (bezogen auf die Gesamtmasses des Materials), vorzugsweise im Bereich von 2 bis 50 Gew.-%, liegt, das Glas als Netzwerkbildner Siliziumdioxid enthält. Ebenso wie das hochporöse Knochenersatzmaterial kann das Knochenersatzmaterial ferner ein oder mehrere Netzwerkwandleroxide umfassen. Zur Vermeidung von Wiederholungen wird im Hinblick auf die Netzwerkwandleroxide vollumfänglich auf die entsprechenden Ausführungen oben Bezug genommen, die gleichermaßen für das hier beschriebene Knochenersatzmaterial gelten.

Die erfindungsgemäße Glaskeramik als Knochenersatzmaterial ist aus einem vorgenannte hochporösem Knochenersatzmaterial erhältlich, indem die Siliziumdioxid-Xerogelmatrix mit dem Netzwerkwandler, bevorzugt Natriumoxid, in den Glaszustand umgewandelt wird.

Mit diesem Umwandlungsprozess wird aus dem nanoporösem Xerogel ein vollständig verknüpftes Glasnetzwerk, das mit einer Bruchfestigkeit von etwa 300 MPa bis etwa 400 MPa die mechanische Stabilität des Knochenersatzmaterials erhöht. Die Bruchfestigkeit des beschriebenen Knochenersatzmaterials ist von der weiter unten beschriebenen Restporosität abhängig, so dass die theoretischen Werte nicht erreicht werden.

Die Erfindung betrifft somit auch ein Knochenersatzmaterial, bei dem die Glasmatrix Natriumsilikat ist. Es weist vorzugsweise eine mechanische Festigkeit im Bereich von 30 MPa bis 200 MPa, vorzugsweise 50 MPa bis 120 MPa, auf und hat eine Restporosität von 5 bis 35 %, wobei die Poren einen Durchmesser im Bereich von 1 µm bis 200 µm haben.

Die Figur 3 zeigt schematisch die Struktur der Glaskeramik. Die schwarz gezeichneten Calciumphosphatkristallite haben die identische Struktur wie in hochporösem Knochenersatzmaterial, sie liegen aber jetzt in einer Glasmatrix, die grau dargestellt ist. Die Restporosität wurde in der schematischen Abbildung nicht dargestellt.

Der Prozess der Umwandlung von Gel in das Glas ist mit einem Sintern des hochporösen Knochenersatzmaterials verbunden. Die Nanoporosität wird vollständig beseitigt und die beschriebene Porosität im Mikrometerbereich wird verringert, so dass eine Restporosität von 2 bis 35 Vol.-% erhalten bleibt.

Durch den beschriebenen Anteil an Calciumphosphaten in der Glasmatrix ist das Material biokompatibel. Der Prozess der Resorption hat sich jedoch vollständig geändert, da keine Nanoporosität übrig geblieben ist.

Da die Glasmatrix bevorzugt Natriumsilikatglas ist, gehen bei der Anwendung der Glaskeramik als Knochenersatzmaterial die Natriumionen langsam in Lösung, und das Glas wandelt sich wieder in eine gelähnliche Struktur mit Nanoporen um. Die Restporosität im Mikrometerbereich verstärkt diesen Effekt noch. Durch diesen Prozess ist letztendlich eine Resorption dieses Knochenersatzmaterials möglich.

Sofern der Prozess des Übergangs der Xerogelmatrix des beschriebenen hochporösen Knochenersatzmaterials in die Glasmatrix nur partiell erfolgt, ist ein Knochenersatzmaterial erhältlich, das in den mechanischen Eigenschaften und in den Resorptionseigenschaften kontinuierlich zwischen den beiden Extrema, dem hochporösen Knochenersatzmaterial und der Glaskeramik als Knochenersatzmaterial, eingestellt werden kann.

Die Erfindung betrifft (dementsprechend) ein Knochenersatzmaterial, das dadurch gekennzeichnet ist, das kristallines Calciumphosphat in eine Matrix eingebettet ist, wobei die Kristallite eine Größe von etwa 10 nm bis etwa 2000 nm aufweisen, die Matrix aus einem Xerogel und aus einem Glas besteht, der Glasanteil der Matrix zwischen 0 und 100 Vol.-%, vorzugsweise 10 Vol.-% bis 80 Vol.-% und besonders bevorzugt zwischen 60 Vol.-% und 80 Vol.-%, liegt, Xerogel und Glas aus Siliziumdioxid und einem Netzwerkwandler bestehen, bevorzugt in einem Anteil von 0,5 bis 35 Mol.-%, vorzugsweise in einem Anteil von 17 Mol.-% bis 30 Mol.-%, bezogen auf das Siliziumdioxid vor, der Netzwerkwandler bevorzugt Natriumoxid ist und die Matrix im Bereich von 2 bis 80 Gew.-%, vorzugsweise im Bereich von 4 bis 50 Gew.%, des Knochenersatzmaterials liegt.

Der teilweise Übergang vom Xerogel zum Glas ist durch eine Wärmebehandlung realisierbar. Da die Glastemperatur von Natriumsilikatglas in Abhängigkeit vom Natriumgehalt im Bereich von etwa 460°C bis etwa 800°C liegt, ist es klar, dass eine Wärmebehandlung über diesem Temperaturbereich sehr schnell zum Glas führt. Wird eine Temperaturbehandlung etwa 20% bis etwa 5% unter der für die Zusammensetzung festgelegten Glastemperatur durchgeführt, verlangsamt sich der Prozess und benötigt mehrere Stunden und kann zu jedem Zeitpunkt abgebrochen werden.

Eine zweite Möglichkeit, den Übergang vom Xerogel zum Glas nur partiell durchzuführen, besteht in der Verwendung zweier beschriebener Calciumphosphat/Siliziumdioxid Granulate, die sich im Anteil der Netzwerkwandler unterscheiden. Bevorzugt wird ein Granulat ohne Netzwerkwandler (Na₂O) und ein Granulat mit ca. 20 Mol.-% Na₂O bezogen auf das Xerogel gewählt. Aus diesen Granulaten wird nach dem unten beschrieben Verfahren der hochporöse Knochersatzwerkstoff hergestellt. Wird anschließend eine Wärmehandlung bei ca. 520°C durchgeführt, so gehen die Bereiche mit dem Na₂O in den Glaszustand über, die Bereichte ohne Na₂O bleiben im Zustand des Xerogels, da hier Temperaturen von ca. 1000°C benötigt werden.

Bei dem Knochenersatzmaterial handelt es sich gemäß einer besonderen Ausführungsform um einen Formkörper, insbesondere einen Quader, eine Platte, einen Hohlzylinder oder einen Keil.

Gegenstand der Erfindung ist somit auch ein Formkörper aus den beschriebenen hochporösen Knochenersatzmaterial, der auf mindestens einer Seite eine Schicht aus einem vorgenannten Knochenersatzmaterial mit höherer mechanischer Festigkeit, bevorzugt der beschriebenen Glaskeramik, umfasst, wobei in dieser Schicht Löcher mit einem Durchmesser von 0,5 bis 5 Millimeter enthalten sind, die einen Volumenanteil von 5 bis 80 % bezogen auf das Gesamtvolumen der Schicht aufweisen und diese Löcher wiederum mit dem vorgenannten Granulat und/oder mit dem vorgenannten hochporösen Knochenersatzmaterial gefüllt sind.

Bei den Herstellungsverfahren der vorliegend beschriebenen Materialien, die ferner Gegenstand der Erfindung sind, ist der Ausgangspunkt die Herstellung eines Calciumphosphatgranulats, das dadurch gekennzeichnet ist, dass die Kristallite, wie beschrieben, in einer Xerogelmatrix liegen. Ausgehend von diesem Granulat wird der hochporöse Knochenersatzwerkstoff hergestellt, der wiederum Voraussetzung zur Herstellung der Glaskeramik als Knochenersatzwerkstoff ist.

Erfindungsgemäß wird bei der Herstellung des siliziumdioxidhaltigen Granulats die Herstellung des Calciumphosphats über eine Fällungsreaktion, bei der ein sogenannter Schlicker entsteht, mit einem Gelbildungsprozess des Siliziumdioxides verbunden. Nur so kann realisiert werden, dass separate Nanokristallite in eine Xerogelmatrix eingebaut werden können.

Bei den siliziumdioxidhaltigen Calciumphosphat-Granulaten handelt es sich vorzugsweise um Hydroxylapatit/Siliziumdioxid-Granulate, die optional ferner lösliches Calciumphosphat umfassen.

Im allgemeinen erfolgt die Synthese zur Herstellung von Calciumphosphaten und auch insbesondere von Hydroxylapatit in wässriger Lösung (C.P.A.T. Klein, J.M.A. De Blieck-Hogerworst, J.G.C. Wolke, K. De Groot, Biomaterials,11,509(1990)). Die Hydroxylapatitsynthese kann in alkalischen Medium erfolgen und ergibt thermisch stabile phasenreine Kristallite (M. Asada, Y. Miura, A. Osaka, K. Oukami, S. Nakamura, J.Mat.Sci. 23, 3202(1988); S. Lazic, j. Cryst. Growth, 147,147(1995)). Die Hydroxylapatitsynthese in neutraler oder leicht saurer Umgebung ist auch möglich, jedoch schwieriger zu steuern (H.E.L. Madsen, G. Thodvadarson, J. Cryst. Growth, 66,369 (1984)).

Ausgegangen wird z.B. von Calciumnitrat und Ammoniumhydrophosphat mit einem Verhältnis von Calcium zu Phosphat von 10:6, wenn Hydroxylapatit erhalten werden soll (US 5,858,318). Andere Ausgangsmaterialien sind NaHCO₃ und CaHPO₄ (Th. Leventouri, H.Y. Moghaddam, N. Papanearchou, C.E. Bunaciu, R.L.Levinson, O. Martinez, Mat. Res. Soc. Symp. Proc. 599, 79 (2000)) oder Ca (H₂PO₄)₂ und CaCl₂ (M.Okido, R. Ichina, K. Kuroda, R. Ohsawa, O. Takai, Mat. Res. Soc. Symp. Proc. 599,153(2000)). Auch hier wird, wenn Hydroxylapatit erhalten werden soll, ein Verhältnis von Calcium zu Phosphor von 1,67 gewählt.

Es ist auch möglich, die Fällungsreaktion mit Kalkmilch und Phosphorsäure durchzuführen (DE 42 32 443 C1, US 4,274,879). Wird z.B. Hydroxylapatit über diese Ausgangsstoffe hergestellt, was wiederum durch das Verhältnis von Calcium zu Phosphor der Ausgangsstoffe steuerbar ist, entsteht häufig Dicalciumphosphat als Nebenprodukt, was unerwünscht ist. Es ist also von Vorteil, von rein löslichen Ausgangsstoffen auszugehen und nicht Kalkmilch (eine Dispersion) zu verwenden.

In der zitierten Literatur ist beschrieben, wie die Parameter pH-Wert, Homogenität des Gemisches der Ausgangsstoffe und Temperatur die Kristallitgröße und den Kristallinitätsgrad der Endprodukte beeinflussen. Insbesondere ist der Zusammenhang zwischen pH-Wert und Temperatur der Lösung von Bedeutung (M.Okido, R. Ichina, K. Kuroda, R. Ohsawa, O. Takai, Mat. Res. Soc. Symp. Proc.599,153 (2000)). Bemerkenswert ist, dass Hydroxylapatit in nahezu allen Lösungen feinkristallin, d.h. als Nanokristallite, ausfällt und für bestimmte Anwendungen, z.B. als Putzkörper bei der Zahnpflege eher nach Verfahrensschritten gesucht wird, die zu größeren Kristalliten führen (DE 42 32 443 C1).

Die Mengen der Ausgangsstoffe werden so gewählt, dass ein Ca/P Verhältnis von 1,50 bis 1,67 entsteht. Das Fällungsprodukt ist in diesem Bereich immer sogenanntes "precipitated hydroxyapatite" (PHA. Ca₁₀₋ₓ(HPO₄)ₓ(PO₄)₆₋ₓ(OH)₂₋ₓ. Im Laufe der weiteren Behandlung, die auch Temperaturbehandlungen einschließt, entsteht bei Temperaturen über ca. 650°C aus dem precipitated hydroxyapatite "z.T. vollständig Hydroxylapatit, wenn das Verhältnis von Calcium zu Phosphat (Ca/P-Verhältnis) genau 1,67 ist. Bei einem Ca/P-Verhältnis von 1,5 wird nahezu das gesamte Hydroxylapatit in β-Tricalciumphosphat umgewandelt. Durch ein Ca/P-Verhältnis zwischen 1,5 und 1,67 wird ein Gemische aus β-Tricalciumphosphat und Hydroxylapatit erhalten, dessen endgültige Zusammensetzung durch das Ca/P-Verhältnis eingestellt wird. Bevorzugt wird ein Ca/P-Verhältnis von 1,67 gewählt, um vorzugsweise ausschließlich Hydroxylapatit im Granulat zu erhalten. Soll ein lösliches Calciumphosphat (für die in vivo Anwendung gilt der pH-Wert 7) im Granulat enthalten sein, wird ein Ca/P-Verhältnis kleiner 1,67 gewählt, und es entsteht im Laufe des Prozesses das lösliche β-Tricalciumphosphat.

Die Kristalle in der Lösung neigen zum Agglomerieren. Wird der Feststoff im Anschluss an die Fällung isoliert, so ist das Agglomerieren der Kristalle, insbesondere der Nanokristalle, nicht zu vermeiden (DE 42 32 443 C1). Es entstehen also Granulate aus Calciumphosphatkristalliten, aus denen nicht mehr das erfindungsgemäße Granulat, bei dem die Kristallite in einer Xerogelmatrix liegen, erhalten werden kann.

Erfindungsgemäß wird dieses Problem gelöst, indem die Lösung mit dem ausgefällten Calciumphosphat durch Rühren homogenisiert wird und eine hochkonzentrierte Kieselsäurelösung, wobei bevorzugt Orthokieselsäure genutzt wird, zugeführt wird. Bevorzugt wird Tetraethyloxisilan (TEOS) verwendet, das man vollständig hydrolysiert. Dazu wird bevorzugt TEOS und 0,1 molare Salzsäure im bevorzugtem Volumenverhältnis 30:9 unter starkem Rühren bis zur Hydrolyse gemischt. Das für die Hydrolyse notwendige Wasser liefert die Salzsäurelösung.

Das Verhältnis von Calciumphosphat in der ausgefällten Lösung und der zugegebenen Kieselsäure wird so gewählt, dass die erfindungsgemäße Zusammensetzung des Granulats von etwa 2 Gew.-% bis etwa 80 Gew.-% Siliziumdioxid erhalten wird. Insbesondere ist dabei zu beachten, dass aus einem Liter TEOS 270 g Siliziumdioxid entstehen. Soll z.B. ein Granulat erhalten werden, das 30 Gew.-% Siliziumdioxid enthält, so sind für eine Lösung mit 100 g Calciumphosphat 43 g Siliziumdioxid notwendig, was wiederum bedeutet, dass ca. 160 ml TEOS verwendet werden. Das ist unabhängig davon, wie viel Lösungsmittel die ausgefällte Lösung enthält.

Erfindungsgemäß wird nun der pH-Wert des Gemisches aus dem gefällten Calciumphosphat und der Kieselsäure in einem Bereich von 2 bis 8, bevorzugt in einem Bereich von 5 bis 6,5 eingestellt.

Die Kieselsäure im Schlicker beginnt zu kondensieren und damit die Viskosität des Gemisches zu erhöhen. Bis zu einer Viskosität von bevorzugt 2·10⁵ cP wird in dem Gemisch durch Rühren eine Sedimentation des Calciumphosphates verhindert.

Durch die einsetzende Gelbildung des Siliziumdioxides wird das Gemisch fixiert. Die Calciumphosphatkristallite liegen nun in einer Matrix aus einem Siliziumdioxidhydrogel. Durch Entfernen des Lösungsmittels wird aus der Hydrogelmatrix die erfindungsgemäße Xerogelmatrix. Da ein erfindungsgemäßes Granulat eine Granulatkorngröße von etwa 1 µm bis etwa 1000 µm hat, ist ein Zerkleinern notwendig. Diese Zerkleinerung erfolgt bevorzugt im Zustand des Hydrogels.

Das Hydrogel wird nun in einem abgeschlossenen Gefäß, vorzugsweise bei Raumtemperatur (ggf. auch bei Temperaturen von etwa 60°C bis etwa 80°C), gelagert, bevorzugt über einen Zeitraum von etwa 24 h bis 48 h. Während dieser Zeit findet eine Alterung des Siliziumdioxidgels statt, d.h. in dem festen Gel finden weitere Kondensationsreaktionen statt.

Anschließend wird das Gel mit dem Calciumphosphat getrocknet, um Lösungsmittel zu entfernen. Die Trocknungstemperatur liegt bevorzugt bei etwa 20°C bis etwa 150°C, vorzugsweise wird bei etwa 120°C getrocknet.

Durch ein Ausfrieren des feuchten Hydrogels erhält man erfindungsgemäß ebenfalls ein Calciumphosphat/Siliziumdioxid-Granulat (Hydroxylapatit/Siliziumdioxid-Granulat). Durch die Kristallisation des Wassers werden das Calciumphosphat und das Siliziumdioxid des Hydrogels zusammengedrückt und bilden damit Granulate, die nach dem Auftauen des Eises abfiltriert werden. Die abfiltrierten Granulate werden bevorzugt bei etwa 20°C bis etwa 150°C, vorzugsweise bei etwa 120°C, getrocknet.

Eine besondere Ausführungsform der erfindungsgemäßen Herstellung der Granulate ist dadurch gekennzeichnet, dass das Gemisch aus dem gefällten Calciumphosphat und der Kieselsäure, dessen pH-Wert in einem Bereich von 2 bis 8, bevorzugt in einem Bereich von 5 bis 6,5 eingestellt ist, vor der Gelbildung sprühgetrocknet wird, was den Vorteil hat, dass auf eine einfache Weise Granulatkorngrößen im erfindungsgemäßen Bereich erhältlich sind.

Sprühtrocknen ist ein im Stand der Technik (vgl. z.B. K. Masters, "Spray Drying", 2nd ed., John Wiley&Sons, New York, 1976) bekanntes Verfahren.

Beim Sprühtrocknen werden flüssige Produkte am oberen Ende eines Trockenturms in feine Tröpfchen zerstäubt. Die Tropfen werden während ihres freien Falls durch einen Heißluftstrom im Turm getrocknet. Die Temperatur des Heißluftstroms liegt zwischen etwa 80°C und etwa 200°C und wirkt nur für die Dauer von einer halben bis einer Sekunde auf die Produkte ein. Nach der Gefriertrocknung ist die Sprühtrocknung die zweitschonendste industriell angewandte Trockenmethode, insbesondere in der Lebensmittelindustrie.

Wird durch die einsetzende Kondensation der Kieselsäure eine kinematische Viskosität von bevorzugt 0,5 bis 50 cst erreicht, wird das Gemisch sprühgetrocknet, wobei der Druck so auf die Konzentration und die Viskosität abgestimmt wird, dass Granulate von 10 µm und kleiner entstehen. (Siehe hierzu Masters, Spray Drying Handbook, (1979) Georg Godwin Ltd;).

Durch das Verdunsten des Lösungsmittels wird die Gelbildung erreicht und ein Übergang vom nassen Gel zum Xerogel eingeleitet. Das Sprühtrocknen bewirkt, dass mit Gelbildung der kleinen Tröpfchen und dem Trocknen der kleinen Tröpfchen Granulatkörner entsprechender Größe entstehen.

Das Granulat ist dadurch gekennzeichnet, dass die Calciumphosphat-Kristallite (vorzugsweise HA-Kristallite) durch ein poröses Siliziumdioxidgel zusammengehalten werden.

Eine Charakterisierung der Granulate erfolgt mit Elektronenmikroskopie und mit Photokorrelationspektroskopie. (E.R. Pike and J.B. Abbiss eds. Light Scattering and Photo Correlation Spectroscopy. Kluwer Academic Publisher, 1997).

Eine im Bereich von etwa 200°C bis etwa 800°C liegende Temperaturbehandlung des Granulates, das nach einem der oben beschriebenen Verfahren erhältlich ist, gewährleistet, dass restliches Lösungsmittel aus den Poren beseitigt wird. Dabei ist zu beachten, dass vorhandener Alkohol, soweit als Lösungsmittel verwendet, vor der Temperaturbehandlung möglichst vollständig entfernt wird, da er sonst nachfolgend bei höheren Temperaturen durch Bildung von Kohlenstoff das Produkt verunreinigt.

Eine Temperaturbehandlung bei etwa 700°C bis etwa 900°C bevorzugt (etwa 800°C bei Anwesenheit von Sauerstoff (normale Luftatmosphäre)) entfernt den gegebenenfalls vorhandenen Kohlenstoff durch Oxidation.

Eine besondere Ausführungsform des erfindungsgemäßen Granulats enthält, wie oben beschrieben, 0,5 Mol-% bis 35 Mol% eines Netzwerkwandlers im Xerogel, bevorzugt Na₂O.

Der Netzwerkwandler wird bevorzugt in das fertige nanoporöse Granulat eingebracht, indem bevorzugt eine wässrige Lösung verwendet wird. Ein Trocknungsprozess bei bevorzugt etwa 120°C bis etwa 200°C entfernt anschließend das Lösungsmittel. (Beispiel: Für 100 g eines Granulats mit 30 Gew.-% Siliziumdioxid werden 8 g NaOH in 50 ml destilliertem Wasser gelöst. Das poröse Granulat nimmt diese Lösung auf, und es wird sofort getrocknet, um ein Auflösen der Xerogels in der basischen Lösung zu verhindern.) Im Granulat liegt damit das Netzwerkwandleroxid mit 21 Gew.-% vor, was 19,3 Mol-% Na₂O bezogen auf das Xerogel entspricht.

Die Erfindung betrifft somit auch ein Verfahren zur Herstellung eines erfindungsgemäßen Granulats bei dem unter Verwendung entsprechender Orthophosphat-Verbindungen und Calcium-Verbindungen (wie z.B. Calciumnitrat und Ammoniumhydrophosphat) durch die Reaktion der Orthophosphatgruppe PO₄³⁻ und Calciumionen in wässriger Lösung ein Hydroxylapatit ausgefällt wird, das durch die in der Lösung festgelegten Ionenkonzentrationen ein Ca/P-Verhältnis von 1,50 bis 1,67 aufweist, wobei bevorzugt ein Ca/P-Verhältnis von 1,67 gewählt wird, wenn das Endprodukt ausschließlich als Calciumphosphat Hydroxylapatit enthalten soll, und wobei ein Ca/P-Verhältnis kleiner 1,67 gewählt wird, wenn im Endprodukt zusätzlich das lösliche β-Tricalciumphosphat vorhanden sein soll.

Das Verfahren ist weiterhin dadurch gekennzeichnet, dass das ausgefällte Hydroxylapatit, ohne dass es in der wässrigen Lösung Agglomerate bildet, homogen in ein Siliziumhydrogel eingebettet wird, was man dadurch realisiert, dass man der wässrigen Lösung Kieselsäure, bevorzugt Orthokieselsäure, insbesondere hydrolysiertes Tetraethyloxisilan (TEOS), zuführt und man den pH-Wert so einstellt, dass er im Bereich von 2 bis 8, bevorzugt von 5 bis 6,5 liegt, so dass eine Gelbildung erfolgt. Die verwendete Menge TEOS wird so gewählt, dass der Siliziumdioxid-Anteil im Bereich von 4 bis 80 Gew.-%, vorzugsweise im Bereich von 2 bis 50 Gew.-%, bezogen auf die Gesamtmasse der Granulatkörner, liegt. Durch einen Trocknungsprozess kommt es zu einem Übergang vom Hydrogel zum Xerogel, wodurch die Calciumphosphatkristallite in einer Xerogelmatrix liegen.

Das erfindungsgemäß hergestellte Calciumphosphat-Granulat (unlösliches Calciumphosphat, welches vorzugsweise Hydroxylapatit ist, gegebenenfalls in Kombination mit löslichem Calciumphosphat, bevorzugt β-Tricalciumphosphat, das in definierter Konzentration und Morphologie Siliziumdioxid enthält, dient, wie bereits erwähnt, als Ausgangsmaterial für die Herstellung des hochporösen Knochenersatzmaterials. Das Herstellungsverfahren wird weiter unten beschrieben. Eine Anwendung als Ausgangsmaterial für die Plasmaspraybeschichtung (siehe R. B. Heimann, Plasma-Spray Coatings. Principles and Applications, Wiley-VCH Verlag (1998)) von Implantaten. Hierbei werden die Teile, die direkten Kontakt zum Knochen haben, wie z.B. der Schaft einer Hüftgelenksprothese mit dem Material beschichtet. Eine Anwendung bei Zahnimplantaten ist ebenso möglich.

Wird das Granulat mit Knochenmarkflüssigkeit oder mit Eigenblut des Patienten gemischt, so ist es als injizierbares Arzneimittel bzw. Medizinprodukt anzuwenden, das dazu dient, osteoporotische Knochen aufzubauen, den Knochenaufbau im Übergangsbereich zu gelockerten Metallimplantaten anzuregen oder die Heilung von Parodontaldefekten anzuregen.

Aus dem erfindungsgemäßen Granulat wird der erfindungsgemäße hochporöse Knochersatzwerkstoff hergestellt. Dabei wird ein Schlicker aus den beschriebenen Granulat und bevorzugt Wasser hergestellt. Auf etwa 100 g Granulat werden bevorzugt etwa 100 ml bis etwa 300 ml Wasser gegeben. Nachdem der pH-Wert bevorzugt so eingestellt wird, dass er im Bereich von 5 bis 6,5 liegt, wird der Schlicker in eine beliebige Form gegossen und getrocknet. Damit wird ein hochporöses Knochenersatzmaterial erhalten. Vergleichbar ist der entstandene Formkörper mit einem Grünkörper, wie er üblicherweise bei keramischen Verfahren anfällt (siehe hierzu: D. Richerson, Modern Ceramic Engineering, Dekker Publ., J. Reed, Principles of Ceramic Processing, Nanocrystalline Ceramics, M. Winterer, Springer 2002).

Da bei dem erfindungsgemäßen Calciumphosphatgranulat die Calciumphosphatkristallite in einer Matrix aus Siliziumdioxidxerogel liegen, besteht die Oberfläche der Granulate natürlich aus Silizumdioxid, das in dem gewählten pH-Bereich bestrebt ist, zwischen den -SiOH-Gruppen der Oberflächen sich berührender Granulatkörner eine Kondensationsreaktionen durchzuführen. Durch den Kapillardruck beim Trocknungsprozess werden die Oberflächen der Granulatkörner aufeinander gedrückt und durch -SiOSi-Bindungen verbunden. Dadurch erhält das hochporöse Knochenmaterial seine mechanische Stabilität und die beschriebenen erfindungsgemäßen Eigenschaften. Als zusätzlicher Binder kann Kieselsäure, insbesondere Orthokieselsäure, in den Schlicker gegeben werden. Gemäß einer Ausführungsform der Erfindung wird dazu TEOS mit Salzsäure hydrolysiert und dem Schlicker zugegeben. Dabei werden auf 100 g Granulat bevorzugt 3 ml bis 15 ml TEOS genommen.

Die Trocknung des Schlickers erfolgt bevorzugt bei einer Temperatur zwischen etwa Raumtemperatur und etwa 200°C, besonders bevorzugt zwischen etwa 80°C und etwa 130°C. Nach dem Trocken erfolgt eine weitere Temperaturbehandlung zum Verfestigen des hochporösen Knochenersatzmaterials bei einer Temperatur, die vom Vorhandensein von Netzwerkwandlern im Xerogel des Granulates abhängt. Ohne Netzwerkwandler (reines Siliziumdioxidxerogel) erfolgt die Temperaturbehandlung bevorzugt bei etwa 700°C bis etwa 900°C, vorzugsweise bei ca. 800°C. Mit Netzwerkwandler im Xerogel liegt die Temperatur bevorzugt im Bereich zwischen etwa 300°C und etwa 500°C.

Durch das beschriebene Verfahren erhält das hochporöse Knochenersatzmaterial seine beschrieben Struktur und damit die beschriebenen Eigenschaften.

Zusätzlich zu den Nanoporen im Xerogel entsteht eine Porenkaterorie, die durch die Packung der Granulatkörner und deren Größe bestimmt ist. Eine weitere Porenstruktur im Größenbereich von einigen hundert µm bis in den mm-Bereich, die ein Einwachsen von Blutgefäßen ermöglichen soll, wird im Formkörper erzeugt, indem dem Schlicker zusätzlich bevorzugt organische Pulver mit einer Korngröße in der später gewünschten Porengröße zugegeben werden, die nach dem Trocknungsprozess herausgebrannt werden.

Bevorzugt werden durchgehend Poren (Kanäle) (im Größenbereich von einigen hundert µm bis in den mm-Bereich) erzeugt, indem man organische Fasern des gewünschten Durchmessers in den Schlicker einbringt, die nach dem Trocknungsprozeß herausgebrannt werden.

Als Material für das Pulver oder die Fasern kommt insbesondere Wachs in Frage, da dann eine Trocknung des Materials, die immer eine geringe Schrumpfung mit sich bringt, bei Temperaturen durchgeführt werden kann, wo das Wachs weich ist und damit ein Reißen des Materials verhindert. Eine günstige Trocknungstemperatur ist somit etwa 40°C. Anschließend kann das Wachs durch Zentrifugieren bei ca. 100°C aus den Poren entfernt werden. Reste des Wachses werden anschließend herausgebrannt, und bei etwa 800°C wird der entstehende Kohlenstoff entfernt.

Das Verfahren zur Herstellung der beschriebenen erfindungsgemäßen Glaskeramik geht von dem beschriebenen hochporösen Knochenersatzmaterial aus.

Hierbei wird die Xerogelmatrix des hochporösen Knochenersatzwerkstoff in eine Glasmatrix umgewandelt, ohne dass es zum Zusammensintern der Calciumphosphatkristalle kommt. Das bedeutet, dass die Verknüpfung der Siliziumdioxidtetraeder vervollständigt wird

Ein Gel-Glas-Übergang verlangt bei reinem Siliziumdioxid eine relativ hohe Temperatur von ca. 900°C bis 1200°C. Da bei diesen Temperaturen die Möglichkeit besteht, dass die kristallinen Calciumphosphatkomponenten einen Phasenübergang machen, wird bevorzugt ein hochporöses Knochenersatzmaterial mit einem Netzwerkwandler im Xerogel verwendet. Die Netzwerkwandler sind entweder schon durch die ursprüngliche Verwendung eines Granulates mit einem Netzwerkwandler in das hochporöse Knochenersatzmaterial gelangt, oder die Netzwerkwandler werden in das fertiggestellte hochporöse Knochenersatzmaterial eingebracht, indem man dieselbe Methode wie beim Granulat anwendet. Damit erfolgt ein Gel-Glas-Übergang bei weit geringeren Temperaturen, und die Calciumphosphatkomponente verändert sich nicht. Typische Netzwerkwandlerkonzentrationen liegen bei 0,5 bis 35 Mol-%, vorzugsweise 17 bis 30 Mol-%, bezogen auf den Siliziumdioxidanteil. Als Netzwerkwandleroxid bietet sich Na₂O an, da die Glasphase damit in Körperflüssigkeit löslich ist und damit auch resorbiert werden kann.

Da die Glastemperatur von Natriumsilkatglas in Abhängigkeit vom Natriumgehalt im Bereich von etwa 460°C bis etwa 800°C liegt, ist es klar, dass eine Wärmebehandlung über diesem Temperaturbereich sehr schnell zum Glas führt. Wird eine Temperaturbehandlung etwa 20% bis etwa 5% unter der für die Zusammensetzung festgelegten Glastemperatur durchgeführt, verlangsamt sich der Prozess und benötigt mehrere Stunden und kann zu jedem Zeitpunkt abgebrochen werden.

Während der Resorption geht dann das Glas den umgekehrten Weg. Das heißt, aus dem Glas wird wieder eine gelähnliche Struktur.

Mit den erfindungsgemäßen Calciumphosphat/Siliziumdioxid-Granulaten besteht nun die Möglichkeit, Festigkeit und Resorptionseigenschaften des erfindungsgemäßen Knochenersatzmaterials zu optimieren. Eine Erhöhung der Festigkeit wird auf jeden Fall immer mit einer Verschlechterung der Biodegradation einhergehen.

Mit dem erfindungsgemäßen Knochenersatzmaterial sind viele Anwendungen möglich. Für kleine Defekte, wie sie z.T. in der Kieferchirurgie auftreten, kann ein Granulat aus dem hochporösem Knochenersatzmaterial zum Auffüllen verwendet werden. Bei größeren Defekten, wo der verbleibende Knochen die Form des Defektes noch genügend stabilisiert, sind Formkörper aus dem hochporösen Knochenersatzmaterial anzuwenden.

Insbesondere Formkörper in einer Kombination von mechanisch festeren Knochenersatzmaterialien (Matrix besteht aus Glas) und den hochporöseren Knochenersatzmaterialien (Matrix besteht aus Xerogel) zeigen interessante Anwendung insbesondere bei größeren Defekten oder auch bei Defekten, bei denen kein nativer Knochen als Leitschiene verblieben ist.

Erfindungsgemäß haben diese Formkörper von mindestens einer Seite eine Schicht aus dem anorganisches resorbierbares Knochenersatzmaterial mit dem Glas als Matrix (erhöhte Festigkeit) und das in dieser Schicht Löcher in der Größenordnung von 0,5 bis 5 Millimeter sind und diese Löcher einen Volumenanteil in der Schicht von 5 bis 80% einnehmen. Das gesamte Volumen einschließlich der Löcher in den festeren Material wird durch das Material, das ein Xerogel als Matrix besitzt, eingenommen. Die Löcherstruktur in der festen Schicht soll ein Einwachsen von Blutgefäßen ermöglichen.

Die Erfindung betrifft daher ferner die Verwendung der erfindungsgemäßen Granulate und Knochenersatzmaterialien zur Herstellung von Formkörpern, vorzugsweise eines Quaders, einer Platte, eines Hohlzylinders oder eines Keils.

Ferner ermöglicht die Erfindung die Verwendung des vorgenannten Siliziumdioxid/Calciumphosphat-Granulates zur Beschichtung von Implantaten (s.o.). Besonders bevorzugt erfolgt die Beschichtung durch Plasmaspraybeschichtung.

Die Erfindung betrifft ferner die Verwendung eines erfindungsgemäßen Granulats zur Herstellung eines Arzneimittels oder Medizinprodukts zum Aufbau osteoporotischer Knochen, zum Anregen des Knochenaufbaus im Übergangsbereich zu gelockerten Metallimplantaten oder zum Anregen der Heilung von Parodontaldefekten. Dabei vermischt man das Granulat vorzugsweise mit Knochenmarkflüssigkeit oder Blut.

Gegenstand der Erfindung ist ferner ein Arzneimittel oder Medizinprodukt, das ein erfindungsgemäßes Granulat umfasst, das mit Knochenmarkflüssigkeit oder Blut des Patienten (somit autolog) vermischt ist.

Gegenstand der Erfindung ist ferner ein Arzneimittel oder Medizinprodukt, das ein erfindungsgemäßes hochporöses Knochenersatzmaterial oder eine Glaskeramik als Knochenersatzmaterial umfasst, wobei das Knochenersatzmaterial unmittelbar vor dem Implantieren mit Knochenmarkflüssigkeit oder Blut des Patienten (somit autolog) so in Kontakt gebracht wird, das sich die Poren der Materialien vollständig füllen.

Die vorliegende Erfindung wird nachfolgend anhand von Beispielen und Figuren näher erläutert, ohne darauf beschränkt zu sein.

### BEISPIELE

### Beispiel 1 - Erzeugung von Calciumphosphat-Granulat

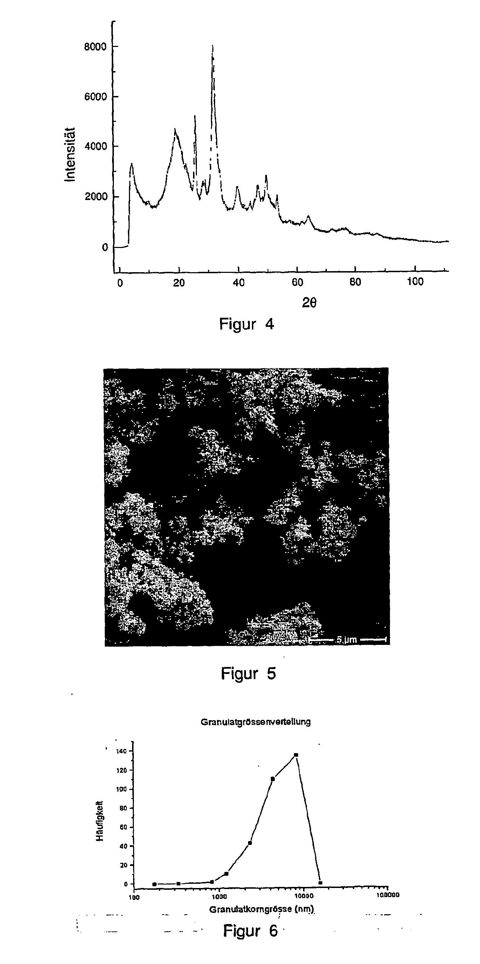

Eine Lösung von 3 mmol/m³ Ca (H₂PO₄)₂ und eine Lösung von 7 mmol/m³ CaCl₂ wird zusammengerührt (womit ein Ca/P Verhältnis von 1,67 erhalten wird) und es wird ein pH-Wert von 7 mit NH4OH eingestellt. Das ausgefällte Material wird mit Pulverdiffraktometrie vermessen. Abbildung 4 zeigt das Ergebnis. Es handelt sich um phasenreines Hydroxylapatit, was sich auch während der folgenden Verfahrensschritte nicht ändert. Die Lösung mit dem ausgefällten Hydroxylapatit wird durch ständiges Rühren am Sedimentieren gehindert und aufkonzentriert, bis auf 100 ml Lösungsmittel 50 g Hydroxylapatit verbleiben. 60 ml Tetraethyloxisilan (TEOS) und 18 ml 0,05 molare Salzsäure werden stark verrührt bis die Hydrolyse des TEOS vollzogen ist, was eine Zeit von ca. 15 Minuten benötigt und durch einen Temperaturanstieg von Raumtemperatur auf ca. 50°C nachweisbar ist.

Diese Lösung wird der Lösung mit dem ausgefällten, homogen verteilten Hydroxylapatit zugeführt und mit NH₄OH der pH Wert auf ca. 6.0 eingestellt. Dieses Gemisch wird weiter gerührt bis eine Viskosität von ca. 2*10^5 cP erreicht wird (durch die einsetzende Gelbildung des Siliziumdioxides wird die Lösung pastenartig). Nach der sofort einsetzenden Gelbildung wird der Ansatz 24 Stunden in einem abgeschlossenen Gefäß gelagert, anschließend granuliert.

Darauf erfolgt die Trocknung bei 80°C über einen Zeitraum von 2 Stunden. Hierbei erfolgt der Übergang vom Hydrogel zum Xerogel.

Das Granulat wird in destilliertem Wasser gespült und anschließend wieder getrocknet. Hierzu wurde eine Temperaturbehandlung von 120°C bei einer Zeitdauer von zwei Stunden gewählt.

Die anschließende Temperaturbehandlung bei 800°C beansprucht eine Zeit von 1 Stunde. Das entstandene Granulat besteht zu 75 Gew.-% aus Calciumphosphat und zu 25 Gew.-% aus Siliziumdioxid.

Das entstandene Granulat wird durch rasterelektronenmikroskopische Aufnahmen charakterisiert, wie sie in Abbildung 5 zu sehen ist. Es sind Granulatkörner im Größenbereich von 1 µm bis 5 µm zu erkennen.

Aus dem Granulat wird mit Wasser ein Schlicker hergestellt und mit dynamischer Lichtstreuung (E.R. Pike and J.B. Abbiss eds. Light Scattering and Photo Correlation Spectroscopy. Kluwer Academic Publisher, 1997) die Größenverteilung der Granulatkörner bestimmt. Das Ergebnis ist in Abbildung 6 zu sehen.

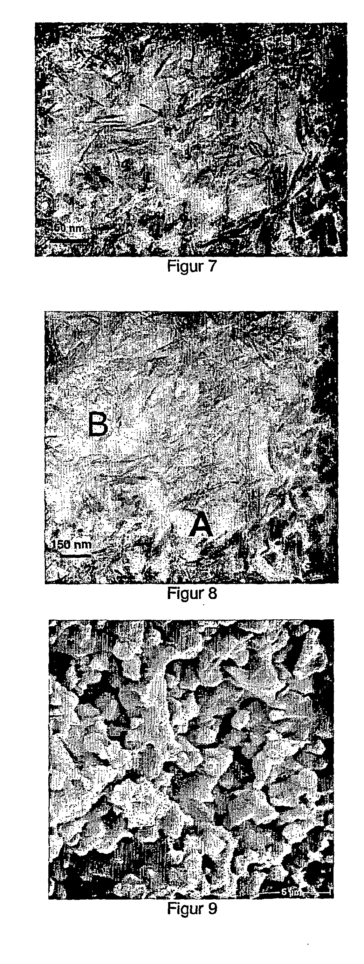

Die Figuren 7 und 8 zeigen transmissionselektronenmikroskopische Aufnahmen von Schnitten durch die Granulatkörner. Hierzu wurde das Material in Epoxid eingebettet und es wurden ca. 60 nm dicke Schnitte angefertigt. Die Kristallite sind Plättchen mit einem mittleren Plättchendurchmesser von 150 nm und einer Plattchendicke von ca. 10-20 nm. Es ist sehr schön zu erkennen, wie die Kristallite in der Xerogelmatrix eingebettet sind, obwohl der Kontrastunterschied zwischen dem Epoxid (Einbettungsmaterial) und dem Siliziumdioxidxerogel nur relativ schwach ist. In Figur 7 ist z.B. die Region A eine mit Epoxid gefüllte Pore und Region B ein typisches Gebiet in dem das Hydroxylapatit im Xerogel eingebettet ist.

### Beispiel 2 - Erzeugung von Calciumphosphat-Granulat

Eine wässrige Lösung von Calciumnitrat und Ammonium Hydrophosphat mit einem Calcium zu Phosphatverhältnis von 1.67 wird mit einem Magnetrührer homogen vermischt und ein pH-Wert von 10 mit Hilfe von NH₄OH eingestellt. Das ausgefällte Material wird viermal mit destilliertem Wasser gewaschen und zentrifugiert und anschließend in Ethanol dispergiert.

Bezogen auf einen Feststoffanteil von 72,9g HA wird 30 ml TEOS mit 9ml einer 0,1 mol/l HCl-Lösung und 9ml Ethanol gemischt. Nach der Hydrolyse des TEOS wird dieses Gemisch in den HA Schlicker gegeben und homogen verteilt und ein pH-Wert von 6.0 eingestellt.

Das Sprühtrocknen erfolgt, indem der homogenisierte Schlicker mit Pressluft und einem Druck zwischen 50 und 100 kPa durch eine Düse gedrückt wird und das schnelle Trocknen in einem koaxialen Luftstrom bei einer Temperatur von 100°C geschieht.

Die anschließende Temperaturbehandlung bei 800°C beansprucht eine Zeit von 1 Stunde.

Das entstandene Granulat unterscheidet sich in den Eigenschaften von dem Granulat in erster Linie durch die Größe der Granulatkörner, die einen wesentlich schmalere Verteilung und ein Maximum bei einem Durchmesser von 18 µm besitzt.

### Beispiel 3 - Erzeugung von Calciumphosphat-Granulat

Eine wässrige Lösung von 0.3 M Orthophosphorsäure (H₃PO₄) wird mit einer wässrigen Suspension von 0.1 M Calciumhydroxyd (Ca(OH)₂) bei Raumtemperatur vermischt. Damit wird ein Ca/P Verhältnis von 1.5 erhalten. Ein pH-Wert von 10 wird mit NH₄OH eingestellt. Das ausgefällte Material wird viermal mit destilliertem Wasser gewaschen und zentrifugiert und anschließend in Wasser dispergiert, so dass auf 100 ml Lösungsmittel 50 g Calciumphosphat verbleiben. 30 ml TEOS und 9 ml 0,05 molare Salzsäure werden stark verrührt bis die Hydrolyse des TEOS vollzogen ist, was eine Zeit von ca. 15 Minuten benötigt und durch einen Temperaturanstieg von Raumtemperatur auf ca. 50°C nachweisbar ist.

Diese Lösung wird der Lösung mit dem ausgefällten, homogen verteilten Hydroxylapatit zugeführt, und mit NH₄OH wird der pH-Wert auf ca. 6,0 eingestellt. Dieses Gemisch wird weiter gerührt bis eine Viskosität von ca. 2·10⁵ cP erreicht wird (durch die einsetzende Gelbildung des Siliziumdioxides wird die Lösung pastenartig). Nach der sofort einsetzenden Gelbildung wird der Ansatz 24 Stunden in einem abgeschlossenen Gefäß gelagert, anschließend granuliert.

Darauf erfolgt die Trocknung bei 80°C über einen Zeitraum von 2 Stunden. Hierbei erfolgt der Übergang vom Hydrogel zum Xerogel.

Das Granulat wird in destilliertem Wasser gespühlt und anschließend wieder getrocknet. Hierzu wurde eine Temperaturbehandlung von 120°C bei einer Zeitdauer von zwei Stunden gewählt.

Die anschließende Temperaturbehandlung bei 800°C beansprucht eine Zeit von 1 Stunde. Das entstandene Granulat besteht zu 86 Gew.-% aus Calciumphosphat und zu 14 Gew.-% aus Siliziumdioxid.

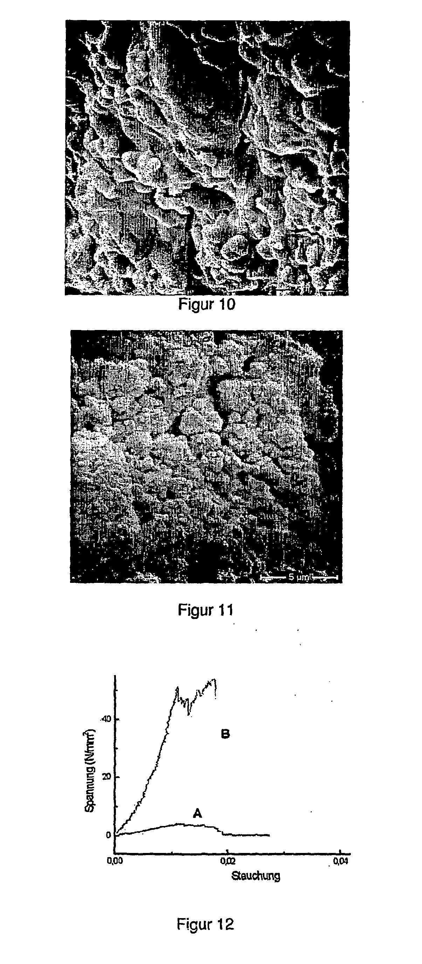

Die Figuren 9 und 10 zeigen rasterelektronenmikroskopische Aufnahmen eines Granulatkornes. In Abbildung 9 ist das innere an einer Bruchkante eines zermahlenen Granulatkorns zu erkennen. Abbildung 10 zeigt die Oberfläche eines Granulatkorns In diesem Beispiel liegen mit dem β-Tricalciumphosphat relativ große Kristallite mit ca. 1 µm Durchmesser vor. Das Xerogel erscheint in den Aufnahmen als kompaktes Material, was natürlich an der Auflösung der rastermikroskopischen Aufnahmen liegt, die die Porosität der Xerogels nicht vollständig auflösen. Es ist aber sehr gut zu erkennen, wie das Xerogel eine Matrix bildet, in der die Kristallite liegen und das das gesamte Granulatkorn von einer Xerogelschicht umgeben ist.

### Beispiel 4 - Erzeugung des hochporösen Knochenersatzmaterials

100g des Granulates, dessen Herstellung in Beispiel 1 beschrieben ist, das 25 Gew.-% Siliziumdioxid enthält, wird mit 150 ml destilliertem Wasser verrührt und in Formen von jeweils 8 mm·15 mm·30 mm gegossen.

Es folgt eine Trocknung über 3 Stunden bei 80 °C. Bei der anschließenden Temperaturbehandlung werden die Proben 2 Stunden bei 120°C gehalten und anschließen wird die Temperatur auf 800 °C erhöht und eine Stunde gehalten.

Das Knochersatzmaterial hat eine Porosität von ca. 60 %.

Figur 11 zeigt die rasterelektronische Abbildung des Materials. Die Granulatkörner, deren ursprüngliche Gestalt in Figur 5 zu sehen ist, bilden jetzt einen durchgehende 3 dimensionale Struktur mit Poren im Mikrometerbereich.

Die Nanostruktur im Inneren der Granulate ist unverändert.

### Beispiel 5 - Erzeugung des hochporösen Knochenersatzmaterials

142 ml Wasser werden mit 8 ml hydrolysierter TEOS Lösung gemischt. Für die Hydrolyse werden auf 30 ml TEOS 18 ml 0,05 molare Salzsäure gegeben und gerührt bis die Hydrolyse vollzogen ist, was an einer Temperaturerhöhung von Raumtemperatur auf ca. 50°C zu erkennen ist.

100 g Granulat, dessen Herstellung in Beispiel 1 beschrieben ist, werden in dieser Lösung homogen verteilt. Es folgt eine weitere Behandlung wie im Beispiel 4.

Durch das zusätzliche Einbringen des Siliziumdioxides ändert sich die wesentliche Struktur des Materials (Mikrometerporen und Nanometerporen) nicht. Die Granulate sind fester verknüpft, was die Gesamtfestigkeit des hochporösen Knochenersatzmaterial um ca. 50% erhöht.

### Beispiel 6 - Erzeugung des hochporösen Knochenersatzmaterials, aber mit Makroporen

Wachsfäden von 0.2 mm Durchmesser werden vollkommen ungeordnet in die Formen des Beispiel 4 gebracht, so dass sie einen Volumenanteil von 30% des Forminhaltes ausmachen. In diese Formen wird ein Schlicker aus siliziumdioxidhaltigem Calciumphosphatgranulat, wie er in Beispiel 5 beschrieben ist, gegeben. Das Trocknen erfolgt jetzt bei 40°C, da hier die Wachsfäden weich sind und noch nicht flüssig und sich damit nicht in den entstehenden Mikrometerporen verteilen, über einen Zeitraum von 4 Stunden.

Bei einer Tempaturbehandlung bei 800°C über einen Zeitraum von 1 Stunde wird das Wachs herausgebrannt.

Die Makroporen, die an Stelle der Wachsfäden entstanden sind, nehmen ca. 30 Vol.-% ein, so dass eine Gesamtporosität von 72% entstanden ist, denn die Mikrometer- und Nanometerstruktur hat sich gegenüber dem Beispiel 5 oder 6 nicht verändert

### Beispiel 7 - Erzeugung einer Glaskeramik

Ausgangspunkt für die Erzeugung der Klaskeramik als Knochenersatzmaterial ist das hochporöse Knochenersatzmaterial, das im Beispiel 4 hergestellt wurde.

Ein Formkörper aus diesem Material hat eine Dichte von 0.8 g/cm³ und damit eine Porosität von ca. 60%. Ein Volumen von 1000 ml des Formkörpers enthält 200 g Siliziumdioxid. Um den Netzwerkwandler in das Xerogel des Formkörpers mit dem Volumen von 1000 ml zu bringen, werden 50 g NaOH in 600 ml Wasser gelöst und in die Poren des Formkörpers gebracht. Der Formkörper saugt die Lösung vollständig auf, und es erfolgt eine Trocknung bei 120°C. Im Formkörper liegt damit das Netzwerkwandleroxid mit 20 Gew.-% vor, was ca. 19 Mol-% Na₂O bezogen auf das Xerogel entspricht.

Nun erfolgt eine Temperaturbehandlung bei 650°C über zwei Stunden. Damit geht das Xerogel in den Zustand des Glases über. Es entsteht Natriumsilikatglas. Der Formkörper schrumpft und behält eine Restporosität von ca. 30 %.

Mit der Figur 12 wird die mechanische Festigkeit der Knochenersatzmaterialien dokumentiert. Die Kurve A im Spannungs-Stauchungs-Diagramm zeigt das Material mit dem Siliziumxerogel als Matrix. Es handelt sich hierbei um ein Material mit 24 Gewichtsprozent Siliziumdioxid und mit Hydroxylapatit als kristalline Komponente.

Die Kurve B in dem Diagramm repräsentiert ein Material identischer Zusammensetzung, wobei hier die Xerogelmatrix in ein Glas überführt wurde. Die Bruchfestigkeit hat sich von ca. 3 auf 50 MPa erhöht.

### Beispiel 7 - In vivo-Testung des hochporösen Knochenersatzmaterials

Göttinger Minischweine wurden für die Tierexperimente genutzt, um die Eigenschaften des Materials als Knochenersatz zu testen. Die Tiere waren adult (ein Jahr alt) und hatten ein Gewicht zwischen 25 und 30 kg. Die Knochendefekte überschritten die kritische Größe von 5 cm³; ihre Abmessungen betragen ca. 3,0 cm · 1,5 cm · 1,5 cm. Sie wurden in den Unterkiefer gesetzt, komplett mit dem Knochenersatzmaterial gefüllt und wieder mit der Knochenhaut geschlossen. Nach 8 Monaten wurden die Schweine getötet, und die Unterkiefer entnommen und röntgenologische, histologische und rastermikroskopische Untersuchungen durchgeführt.

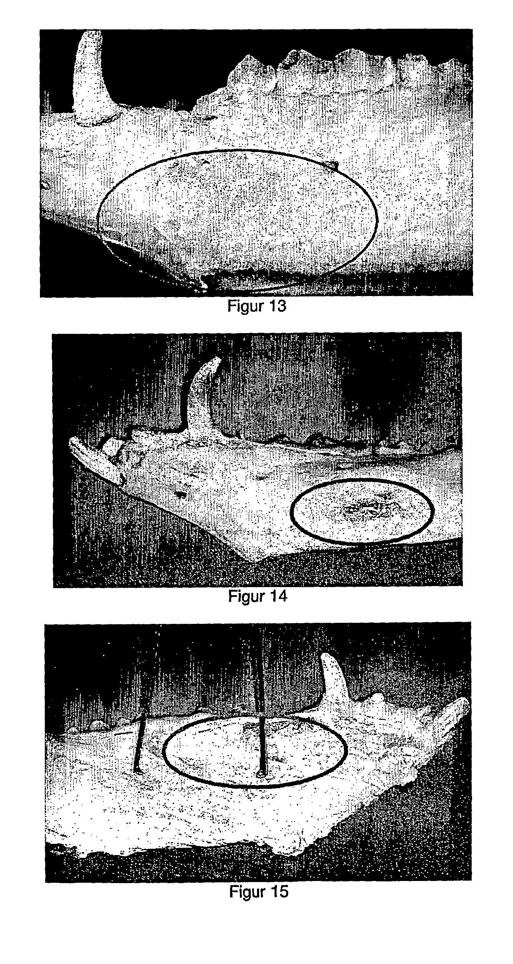

Figur 13 zeigt den Unterkiefer mit dem ehemaligen Defekt, der mit dem Material des Beispiels gefüllt wurde 8 Monate nach der Operation. Das Defektgebiet ist klinisch vollkommen verheilt. Histologische Untersuchungen zeigen, dass über mehrere Versuchstiere gemittelt weniger als 1% des Biomaterials im Defektgebiet vorzufinden ist.

Figur 14 zeigt eine Vergleichsstudie mit einem Leerdefekt. Dieser Defekt wird mit Bindegewebe eingekapselt und verheilt nicht.

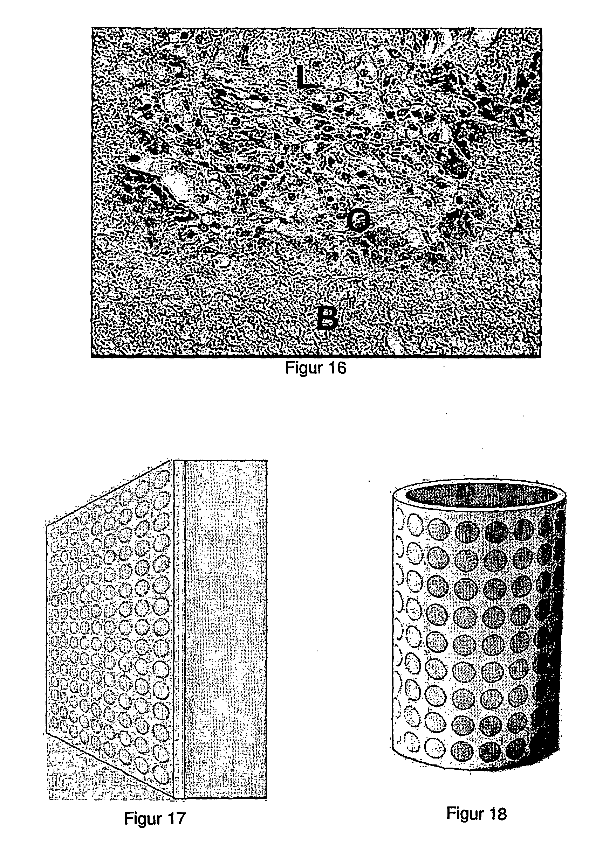

Figur 15 zeigt eine Vergleichsstudie mit einem kommerziellen Knochenersatzmaterial auf Hydroxylapatitbasis. Der Defekt verheilt zwar, aber das Biomaterial wird nicht abgebaut und verbleibt als Fremdkörper im Knochen.

Figur 16 zeigt eine lichtmikroskopische Aufnahme eines histologischen Schnittes. Es handelt sich um ein demineralisierten histologischen Schnitt mit Hämalaun Eosin Färbung. Es ist eine Lagune (L) im Biomaterial des Beispiels (B) zu erkennen. Auf dem Boden der Lagune sind Osteoklasten (O) dabei das Biomaterial abzubauen. Das bedeutet die Biodegradation des Materials erfolgt über Osteoklasten, was für eine Anwendung von entscheidender Bedeutung ist.

### Beispiel 8

In Figur 17 ist ein Formkörper dargestellt, der die Eigenschaft der beiden Materialien mit unterschiedlichen mechanischen Eigenschaften kombiniert und für größere Knochendefekte vorgesehen ist. Das Material mit dem Glas als Matrix bildet auf einer Seite eine Stützschicht, die eine Dicke in der Größenordnung von zwei Millimetern hat, die wiederum mit einem System von Löchern versehen ist. Das Volumen des Formkörpers als auch die Löcher in der stabilen Schicht werden durch das Material mit dem Xerogel als Matrix ausgefüllt, da dieses Material die besseren bioaktiven Eigenschaften hat. Figur 18 zeigt einen weiteren möglichen Formkörper. Der Zylinder hat einen Mantel aus dem Material mit dem Glas als Matrix. Dieser Mantel hat ebenfalls ein System von Löchern, die wie das gesamte Volumen mit dem Material mit dem Xerogel als Matrix gefüllt sind.

## Patentansprüche

1. Granulat auf Basis von Calciumphosphat, **dadurch gekennzeichnet, dass** kristallines Calciumphosphat in einer Siliziumdioxid-Xerogelmatrix eingebettet ist, erhältlich durch Herstellung des Calciumphosphats über eine Fällungsreaktion, bei der man die Lösung mit dem ausgefällten Calciumphosphat durch Rühren homogenisiert, man eine hochkonzentrierte Kieselsäurelösung zugibt, man das Gemisch durch die anschließend einsetzende Gelbildung fixiert und man es durch Entfernen des Lösungsmittels in eine Xerogelmatrix überführt, wobei die in der Xerogelmatrix liegenden Calciumphosphatkristallite eine Größe von etwa 10 nm bis etwa 2000 nm und die Granulatkörner eine Größe von 1 µm bis 1000 µm aufweisen und der Siliziumdioxid-Anteil im Bereich von 2 bis 80 Gew.-%, vorzugsweise im Bereich von 4 bis 50 Gew.-%, bezogen auf die Gesamtmasse der Granulatkörner liegt.

2. Granulat nach Anspruch 1, **dadurch gekennzeichnet, dass** die Poren im Xerogel einen durchschnittlichen Durchmesser im Bereich von 0,5 nm bis 20 nm aufweisen.

3. Granulat nach Anspruch 1 oder 2, **dadurch gekennzeichnet, dass** die Poren in den Granulatkörnern jeweils 10 Vol.-% bis 60 Vol.-% bezogen auf das Volumen des Granulatkorns ausmachen.

4. Granulat nach den Ansprüchen 1 bis 3, **dadurch gekennzeichnet, dass** das Calciumphosphat Hydroxylapatit ist.

5. Granulat nach den Ansprüchen 1 bis 4, **dadurch gekennzeichnet, dass** das Granulat ferner lösliches Calciumphosphat umfasst.

6. Granulat nach Anspruch 5, **dadurch gekennzeichnet, dass** das lösliche Calciumphosphat in einem Anteil von etwa 5 Gew.-% bis 50 Gew.-% bezogen auf den Calciumphosphatanteil vorliegt.

7. Granulat nach Anspruch 5 oder 6, **dadurch gekennzeichnet, dass** das lösliche Calciumphosphat β-Tricalciumphosphat ist.

8. Granulat nach den Ansprüchen 1 bis 7, **dadurch gekennzeichnet, dass** es ferner ein oder mehrere Netzwerkwandleroxide umfasst.

9. Granulat nach Anspruch 8, **dadurch gekennzeichnet, dass** das (die) Netzwerkwandleroxid(e) in einem Anteil von 0,5 bis 35 Mol.-%, vorzugsweise in einem Anteil von 17 bis 30 Mol.-%, bezogen auf das Siliziumdioxid vorliegt (vorliegen).

10. Granulat nach Anspruch 9 oder 10, **dadurch gekennzeichnet, dass** das Netzwerkwandleroxid Na₂O ist.

11. Hochporöses Knochenersatzmaterial, **dadurch gekennzeichnet, dass** er Granulatkörner des Granulats nach den Ansprüchen 1 bis 10 umfasst, die eine dreidimensionale Struktur bilden, die neben den in den Granulatkörnern vorhandenen Poren ferner Poren etwa in der Größe der Granulatkörner aufweist.

12. Knochenersatzmaterial nach Anspruch 11, **dadurch gekennzeichnet, dass** es interkonnektierende Makroporen im Bereich von etwa 100 µm bis zu mehreren 1000 µm aufweist.

13. Knochenersatzmaterial nach Anspruch 11 oder 12, **dadurch gekennzeichnet, dass** es eine Gesamtporosität von 30 bis 90 Vol.-%, vorzugsweise von 60 bis 80 Vol.-% aufweist.

14. Knochenersatzmaterial nach den Ansprüchen 11 bis 13, **dadurch gekennzeichnet, dass** es eine Bruchfestigkeit von etwa 0,1 MPa bis zu 15 MPa, vorzugsweise 3 MPa bis 6 MPa, aufweist.

15. Knochenersatzmaterial nach den Ansprüchen 11 bis 13, **dadurch gekennzeichnet, dass** es ferner ein oder mehrere Netzwerkwandleroxide umfasst.

16. Knochenersatzmaterial nach Anspruch 15, **dadurch gekennzeichnet, dass** das (die) Netzwerkwandleroxid(e) in einem Anteil von 0,5 bis 35 Mol.-%, vorzugsweise in einem Anteil von 17 bis 30 Mol.-%, bezogen auf das Siliziumdioxid vorliegt (vorliegen).

17. Knochenersatzmaterial nach Anspruch 15 oder 16, **dadurch gekennzeichnet, dass** das Netzwerkwandleroxid Na₂O ist.

18. Knochenersatzmaterial, **dadurch gekennzeichnet, dass** es eine Glasmatrix umfasst, in die kristallines Calciumphosphat eingebettet ist, erhältlich durch Herstellung des Calciumphosphats über eine Fällungsreaktion, bei der man die Lösung mit dem ausgefällten Calciumphosphat durch Rühren homogenisiert, man eine hochkonzentrierte Kieselsäurelösung zugibt, man das Gemisch durch die anschließend einsetzende Gelbildung fixiert, man es durch Entfernen des Lösungsmittels in eine Xerogelmatrix überführt, und man anschließend die Xerogelmatrix mit einem Netzwerkwandler in den Glaszustand umwandelt, wobei die Kristallite eine Größe van 10 nm bis 2000 nm aufweisen und der Siliziumdioxid-Anteil im Bereich von 2 bis 80 Gew.-%, vorzugsweise im Bereich von 4 bis 50 Gew.-%, bezogen auf die Gesamtmasse des Knochenersatzmaterials liegt.

19. Knochenersatzmaterial nach Anspruch 18, **dadurch gekennzeichnet, dass** es ferner ein oder mehrere Netzwerkwandleroxide umfasst.

20. Knochenersatzmaterial nach Anspruch 19, **dadurch gekennzeichnet, dass** das (die) Netzwerkwandleroxid (e) in einem Anteil von 0,5 bis 35 Mol.-%, vorzugsweise in einem Anteil von 17 bis 30 Mol.-%, bezogen auf das Siliziumdioxid vorliegt (vorliegen).

21. Knochenersatzmaterial nach Anspruch 19 oder 20, **dadurch gekennzeichnet, dass** das Netzwerkwandleroxid Na₂O ist.

22. Knochenersatzmaterial nach den Ansprüchen 18 bis 21, **dadurch gekennzeichnet, dass** es aus einem Knochenersatzmaterial nach den Ansprüchen 11 bis 17 erhältlich ist, bei dem die Siliziumdioxid-Xerogelmatrix teilweise bis vollständig in den Glaszustand umgewandelt wurde, wobei der Glasanteil der Matrix zwischen 0 und 100 Vol.-%, vorzugsweise 10 bis 80 Vol.-% und besonders bevorzugt zwischen 60 Vol.-% und 80 Vol.-%.

23. Knochenersatzmaterial nach Anspruch 21 und 22, **dadurch gekennzeichnet, dass** die Glasmatrix Natriumsilikat ist.

24. Knochenersatzmaterial nach den Ansprüchen 18 bis 23, **dadurch gekennzeichnet, dass** es eine mechanische Festigkeit im Bereich von 30 MPa bis 200 MPa, vorzugsweise 50 MPa bis 120 MPa, aufweist.

25. Knochenersatzmaterial nach den Ansprüchen 18 bis 23, **dadurch gekennzeichnet, dass** es ein Formkörper ist.

26. Knochenersatzmaterial nach Anspruch 25, **dadurch gekennzeichnet, dass** der Formkörper ein Quader, eine Platte, ein Hohlzylinder oder ein Keil ist.

27. Formkörper aus dem Knochenersatzmaterial nach den Ansprüchen 11 bis 17, **dadurch gekennzeichnet, dass** er auf mindestens einer Seite eine Schicht aus einem Knochenersatzmaterial nach den Ansprüchen 18 bis 24 umfasst, wobei in dieser Schicht Löcher mit einem Durchmesser von 0,5 bis 5 Millimeter enthalten sind, die einen Volumenanteil von 5 bis 80 % bezogen auf das Gesamtvolumen der Schicht aufweisen und diese Löcher wiederum mit dem Granulat nach den Ansprüchen 1 bis 10 und/oder mit dem Knochenersatzmaterial nach den Ansprüchen 11 bis 17 gefüllt sind.

28. Verwendung eines Granulates nach den Ansprüchen 1 bis 10 zur Herstellung von Formkörpern.

29. Verwendung nach Anspruch 28, **dadurch gekennzeichnet, dass** der Formkörper ein Quader, eine Platte, ein Hohlzylinder oder ein Keil ist.

30. Verwendung eines Granulates nach den Ansprüchen 1 bis 10 zur Beschichtung von Implantaten.

31. Verwendung nach Anspruch 30, **dadurch gekennzeichnet, dass** die Beschichtung eine Plasmaspraybeschichtung ist.

32. Verwendung von Granulat nach den Ansprüchen 1 bis 10 zur Herstellung eines Arzneimittels oder Medizinprodukts zum Aufbau osteoporotischer Knochen, zum Anregen des Knochenaufbaus im Übergangsbereich zu gelockerten Metallimplantaten oder zum Anregen der Heilung von Parodontaldefekten.

33. Verwendung nach Anspruch 32, **dadurch gekennzeichnet, dass** man das Granulat durch Mischen mit Knochenmarkflüssigkeit oder Eigenblut des Patienten und gegebenenfalls mit physiologischer Kochsalzlösung herstellt.

34. Arzneimittel oder Medizinprodukt, **dadurch gekennzeichnet, dass** es Granulat nach den Ansprüchen 1 bis 10 umfasst, das mit Knochenmarkflüssigkeit oder Blut eines Patienten vermischt ist.

35. Verfahren zur Herstellung eines Granulats nach den Ansprüchen 1 bis 10, **dadurch gekennzeichnet, dass** man in wässriger Lösung ein Hydroxylapatit ausfällt, das durch die in der Lösung festgelegten Ionenkonzentrationen ein Ca/P-Verhältnis von 1,50 bis 1,67 aufweist, wobei man den pH-Wert, die Homogenität des Gemisches der Ausgangsstoffe und die Temperatur nach bekannten Methoden so einstellt, dass sich Kristallite in der Größe von 10 nm bis 2000 nm und Granulatkörner mit einer Größe von 1 µm bis 1000 µm bilden, wobei das ausgefällte Hydroxylapatit, ohne dass es in der wässrigen Lösung Agglomerate bildet, homogen in ein Siliziumhydrogel eingebettet wird, indem man der wässrigen Lösung Kieselsäure, bevorzugt Orthokieselsäure, zuführt und man den pH-Wert so einstellt, dass er im Bereich von 2 bis 8, bevorzugt von 5 bis 6,5, liegt, so dass eine Gelbildung erfolgt, wobei man die verwendete Menge an Kieselsäure so wählt, dass der Siliziumdioxid-Anteil im Bereich von 2 bis 80 Gew. -%, vorzugsweise im Bereich von 4 bis 50 Gew.-%, bezogen auf die Gesamtmasse der sich bildenden Granulatkörner, liegt, und wobei man das gebildete Hydrogel granuliert und anschließend zur Bildung eines Xerogels einem Trocknungsprozess unterzieht, wodurch die Calciumphosphatkristallite in einer Xerogelmatrix liegen.

36. Verfahren nach Anspruch 35, **dadurch gekennzeichnet, dass** man das Hydrogel in einem abgeschlossenen Gefäß vorzugsweise bei Raumtemperatur und gegebenenfalls auch bei Temperaturen von etwa 60°C bis etwa 80°C bevorzugt über einen Zeitraum von etwa 24 bis 48 Stunden lagert.

37. Verfahren nach Anspruch 35 und 36, **dadurch gekennzeichnet, dass** man die Trocknung des Hydrogels bevorzugt bei einer Temperatur von 20°C bis etwa 150°C, vorzugsweise bei etwa 120°C, durchgeführt.

38. Verfahren zur Herstellung eines Granulats nach den Ansprüchen 1 bis 10, **dadurch gekennzeichnet, dass** man in wässriger Lösung ein Hydroxylapatit ausfällt, das durch die in der Lösung festgelegten Ionenkonzentrationen ein Ca/P-Verhältnis von 1,50 bis 1,67 aufweist, wobei man den pH-Wert, die Homogenität des Gemisches der Ausgangsstoffe und die Temperatur nach bekannten Methoden so einstellt, dass sich Kristallite in der Größe von etwa 10 nm bis etwa 2000 nm und Granulatkörner mit einer Größe von etwa 1 µm bis etwa 1000 µm bilden, wobei das ausgefällte Hydroxylapatit, ohne dass es in der wässrigen Lösung Agglomerate bildet, homogen in ein Siliziumhydrogel eingebettet wird, indem man der wässrigen Lösung Kieselsäure, bevorzugt Orthokieselsäure, zuführt und man den pH-Wert so einstellt, dass er im Bereich von 2 bis 8, bevorzugt von 5 bis 6,5, liegt, so dass eine Gelbildung erfolgt, wobei man die verwendete Menge an Kieselsäure so wählt, dass der Siliziumdioxid-Anteil im Bereich von 2 bis 80 Gew.-%, vorzugsweise im Bereich von 4 bis 50 Gew.-%, bezogen auf die Gesamtmasse der sich bildenden Granulatkörner, liegt, und wobei man wobei vor der Gelbildung eine Spühtrocknung durchgeführt.

39. Verfahren zur Herstellung eines Granulats nach den Ansprüchen 1 bis 10, **dadurch gekennzeichnet, dass** man in wässriger Lösung ein Hydroxylapatit ausfällt, das durch die in der Lösung festgelegten Ionenkonzentrationen ein Ca/P-Verhältnis von 1,50 bis 1,67 aufweist, wobei man den pH-Wert, die Homogenität des Gemisches der Ausgangsstoffe und die Temperatur nach bekannten Methoden so einstellt, dass sich Kristallite in der Größe von 10 nm bis 2000 nm und Granulatkörner mit einer Größe von 1 µm bis 1000 µm bilden, wobei das ausgefällte Hydroxylapatit, ohne dass es in der wässrigen Lösung Agglomerate bildet, homogen in ein Siliziumhydrogel eingebettet wird, indem man der wässrigen Lösung Kieselsäure, bevorzugt Orthokieselsäure, zuführt und man den pH-Wert so einstellt, dass er im Bereich von 2 bis 8, bevorzugt von 5 bis 6,5, liegt, so dass eine Gelbildung erfolgt, wobei man die verwendete Menge an Kieselsäure so wählt, dass der Siliziumdioxid-Anteil im Bereich von 2 bis 80 Gew.-%, vorzugsweise im Bereich von 4 bis 50 Gew.-%, bezogen auf die Gesamtmasse der sich bildenden Granulatkörner, liegt, und wobei man das sich bildende Hydrogel auf Temperaturen unterhalb des Gefrierpunktes des Lösungsmittel abkühlt und die sich bildenden Siliziumdioxid/Hydroxylapatitgranulate nach dem Auftauen abfiltert.

40. Verfahren nach den Ansprüchen 35, 38 oder 39, **dadurch gekennzeichnet, dass** man zum Ausfällen von Hydroxylapatit Calciumnitrat und Ammoniumhydrophosphat mit einem Verhältnis von Calcium zu Phosphat (Ca/P-Verhältnis) von 1,67 verwendet, wobei man den pH-Wert auf etwa 7 bis etwa 10 einstellt.

41. Verfahren nach den Ansprüchen 35, 38 oder 39, **dadurch gekennzeichnet, dass** man zum Ausfällen von Hydroxylapatit Calciumnitrat und Ammoniumhydrophosphat mit einem Verhältnis von Calcium zu Phosphat (Ca/P-Verhältnis) von 1,67 wählt, man ein Ca/P-Verhältnis von kleiner 1,67 jedoch größer 1,50 wählt, wenn das Granulat zusätzlich lösliches β-Tricalciumphosphat umfassen soll, und man ein Ca/P-Verhältnis von 1,50 wählt, wenn das Granulat nur lösliches β-Tricalciumphosphat umfassen soll, und wobei man den pH-Wert auf 7 bis 10 einstellt.

42. Verfahren nach den Ansprüchen 35 bis 41, **dadurch gekennzeichnet, dass** die Kieselsäure hydrolysiertes Tetraethyloxisilan (TEOS) ist.

43. Verfahren nach den Ansprüchen 35 bis 42, **dadurch gekennzeichnet, dass** man den Druck so auf die Konzentration und die Viskosität abstimmt, dass Granulate von 10 µm und kleiner entstehen.

44. Verfahren nach Anspruch 43, **dadurch gekennzeichnet, dass** die kinematische Viskosität 0,5 bis 50 cst beträgt.

45. Verfahren zur Herstellung eines Knochenersatzmaterials nach den Ansprüchen 11 bis 17, **dadurch gekennzeichnet, dass** man zunächst das Verfahren nach den Ansprüchen 35 bis 44 durchführt und man das erhaltene Granulat mit Wasser zu einem Schlicker anrührt, wobei bevorzugt auf 100 g Granulat etwa 100 bis 300 ml Wasser gegeben werden, man anschließend einen pH-Wert von bevorzugt zwischen etwa 2 und 8, besonders bevorzugt zwischen etwa 5 und 6,5, einstellt, man den Schlicker in eine beliebige Form gießt und trocknet, wobei das Trocknen bevorzugt bei einer Temperatur zwischen Raumtemperatur und 200°C, besonders bevorzugt bei einer Temperatur zwischen 80°C und 130°C, erfolgt.

46. Verfahren nach Anspruch 45, **dadurch gekennzeichnet, dass** man dem Schlicker, Kieselsäure, bevorzugt Orthokieselsäure zugibt, so dass bevorzugt auf 100 g Granulat 1 g bis 4 g Kieselsäure kommen.

47. Verfahren nach Anspruch 46, **dadurch gekennzeichnet, dass** die Kieselsäure hydrolysiertes Tetraethyloxisilan (TEOS) ist.

48. Verfahren nach den Ansprüchen 45 bis 47, **dadurch gekennzeichnet, dass** man eine weitere Porenstruktur im Größenbereich von einigen hundert µm bis in den mm-Bereich erzeugt, indem man dem Schlicker zusätzlich organische Pulver mit einer Korngröße in der später gewünschten Porengröße zugibt, die nach dem Trocknungsprozess herausgebrannt werden.

49. Verfahren nach den Ansprüchen 45 bis 47, **dadurch gekennzeichnet, dass** man eine weitere Porenstruktur in Form durchgehend Poren (Kanäle) im Größenbereich von einigen hundert µm bis in den mm-Bereich erzeugt, indem man dem Schlicker zusätzlich organische Fasern des gewünschten Durchmessers zugibt, die nach dem Trocknungsprozess herausgebrannt werden.

50. Verfahren nach den Ansprüchen 48 und 49, **dadurch gekennzeichnet, dass** das Material für das Pulver oder die Fasern Wachs ist.

51. Verfahren nach Anspruch 50, **dadurch gekennzeichnet, dass** man die Trocknung des Knochenersatzmaterials bei etwa 40°C durchführt, man das Wachs optional durch Zentrifugieren bei ca. 100°C aus den Poren entfernt, man anschließend Reste des Wachses herausbrennt und man bei etwa 800°C den entstehenden Kohlenstoff entfernt.

52. Verfahren nach den Ansprüchen 45 bis 51, **dadurch gekennzeichnet, dass** man eine Temperaturbehandlung von etwa 700°C bis etwa 900°C, wenn im verwendeten Granulat keine Netzwerkwandler vorhanden sind, oder von etwa 300°C bis etwa 500°C, wenn im Granulat Netzwerkwandler vorhanden sind, durchführt.

53. Verfahren nach den Ansprüchen 45 bis 52, **dadurch gekennzeichnet, dass** in die Poren des Knochenersatzmaterials eine Lösung mit Netzwerkwandlern gebracht wird, so dass nach Trocknen der Lösung das Netzwerkwandleroxid einem Anteil von 0,5 bis 35 Mol-%, vorzugsweise in einem Anteil von 17 bis 30 Mol-%, bezogen auf das Siliziumdioxid vorliegt.

54. Verfahren zur Herstellung eines Knochenersatzmaterials nach den Ansprüchen 18 bis 26, **dadurch gekennzeichnet, dass** man ein Verfahren nach den Ansprüchen 45 bis 53 durchführt, wobei entweder im verwendeten Granulat Netzwerkwandleroxid enthalten ist und/oder man durch das Verfahren nach Anspruch 46 ein Netzwerkwandleroxid in das Material eingebringt, wobei das Netzwerkwandleroxid insgesamt in einem Anteil von 0,5 bis 35 Mol.-%, vorzugsweise in einem Anteil von 17 bis 30 Mol.-%, bezogen auf das Siliziumdioxid vorliegt und man das erhaltene hochporöse Knochenersatzmaterial einer Temperaturbehandlung von etwa 350°C bis etwa 800°C unterzieht, um die Xerogelmatrix ganz oder teilweise in ein Glas zu überführen.

55. Verfahren nach Anspruch 54, **dadurch gekennzeichnet, dass** man zwei verschiedene Granulate nach den Ansprüchen 1 bis 10 verwendet, die sich im Anteil der Netzwerkwandler unterscheiden, wodurch bei der Temperaturbehandlung der Übergang vom Xerogel zum Glas nur partiell erfolgt.

56. Verfahren nach Anspruch 55, **dadurch gekennzeichnet, dass** man ein Granulat ohne Netzwerkwandler (Na₂O) und ein Granulat mit ca. 20 Mol-% Na₂O bezogen auf das Xerogel verwendet.

57. Verfahren nach Anspruch 56, **dadurch gekennzeichnet, dass** man die Temperaturbehandlung bei etwa 520°C durchführt, wobei die Bereiche mit dem Na₂O in den Glaszustand übergehen, während die Bereiche ohne Na₂O im Zustand des Xerogels verbleiben.

58. Verwendung von hochporösem Knochenersatzmaterial nach den Ansprüchen 11 bis 17 zur Herstellung eines Arzneimittels oder Medizinprodukts zur Auffüllung von kleinen Knochendefekten.

59. Verwendung nach Anspruch 58, **dadurch gekennzeichnet, dass** das Knochenersatzmaterial in Form kleiner Stücke vorliegt.

60. Verwendung nach Anspruch 59, **dadurch gekennzeichnet, dass** die Stücke Zylinder mit einem mittleren Durchmesser von 0,4 bis 2 mm und einer Länge von 1 bis 6 mm sind.

61. Arzneimittel oder Medizinprodukt, **dadurch gekennzeichnet, dass** es ein hochporöses Knochenersatzmaterial nach den Ansprüchen 11 bis 17 oder eine Knochenersatzmaterial nach den Ansprüchen 18 bis 26 umfasst, dessen Poren mit Knochenmarkflüssigkeit oder Blut des zu behandelnden Patienten gefüllt sind.

## Claims

1. Granular material based on calcium phosphate **characterised in that** crystalline calcium phosphate is embedded in a silica xerogel matrix, obtainable by preparing the calcium phosphate via a precipitation reaction, wherein the solution with the precipitated calcium phosphate is homogenised by stirring, a highly concentrated silicic acid solution is added, wherein the mixture is fixed by the subsequently beginning gel formation and transfered into a xerogel matrix by removing the solvent, wherein the calcium phosphate crystallites lying in the xerogel matrix have a size of approximately 10 nm to approximately 2000 nm and the granule grains have a size of 1 µm to 1000 µm, the proportion of silica being in the region of 2 to 80% by weight, preferably in the region of 4 to 50% by weight, based on the total mass of the granule grains.

2. Granular material according to claim 1 **characterised in that** the pores in the xerogel have an average diameter in the region of 0.5 nm to 20 nm.

3. Granular material according to claim 1 or 2 **characterised in that** the pores in the granule grains amount to 10 vol % to 60 vol %, based on the volume of the granule grain.

4. Granular material according to claims 1 to 3 **characterised in that** the calcium phosphate is hydroxyl apatite.

5. Granular material according to claims 1 to 4 **characterised in that** the granular material moreover comprises soluble calcium phosphate.

6. Granular material according to claim 5 **characterised in that** the soluble calcium phosphate is present in a proportion of approximately 5% by weight to 50% by weight, based on the proportion of calcium phosphate.

7. Granular material according to claim 5 or 6 **characterised in that** the soluble calcium phosphate is β-tricalcium phosphate.

8. Granular material according to claims 1 to 7 **characterised in that** it further comprises one or several network modifier oxides.

9. Granular material according to claim 8 **characterised in that** the network modifier oxide(s) is/are present in a proportion of 0.5 to 35 mole %, preferably in a proportion of 17 to 30 mole %, based on the silica.

10. Granular material according to claim 9 or 10 **characterised in that** the network modifier oxide is Na₂O.

11. Highly porous bone substitute material **characterised in that** it comprises granule grains of the granular material according to claims 1 to 10 which form a three-dimensional structure which, apart from the pores present in the granule grains, also exhibits pores with approximately the size of the granule grains.

12. Bone substitute material according to claim 11 **characterised in that** it exhibits interconnecting macropores in the region of approximately 100 µm to several 1000 µm.

13. Bone substitute material according to claim 11 or 12 **characterised in that** it exhibits a total porosity of 30 to 90 vol %, preferably of 60 to 80 vol %.

14. Bone substitute material according to claims 11 to 13 **characterised in that** it exhibits a fracture strength of approximately 0.1 MPa to 15 MPa, preferably 3 MPa to 6 MPa.

15. Bone substitute material according to claims 11 to 13 **characterised in that** it further comprises one or several network modifier oxides.

16. Bone substitute material according to claim 15 **characterised in that** the network modifier oxide(s) is/are present in a proportion of 0.5 to 35 mole %, preferably in a proportion of 17 to 30 mole %, based on the silica.

17. Bone substitute material according to claim 15 or 16 **characterised in that** the network modifier oxide is Na₂O.

18. Bone substitute material **characterised in that** it comprises a glass matrix into which crystalline calcium phosphate is embedded, obtainable by preparing the calcium phosphate via a precipitation reaction, wherein the solution with the precipitated calcium phosphate is homogenised by stirring, a highly concentrated silicic acid solution is added, wherein the mixture is fixed by the subsequently beginning gel formation and transfered into a xerogel matrix by removing the solvent, and wherein the xerogel matrix is subsequently converted into the glassy state with the network modifier, wherein the crystallites have a size of 10 nm to 2000 nm and the proportion of silica being in the region of 2 to 80% by weight, preferably in the region of 4 to 50% by weight, based on the total mass of the bone substitute material.

19. Bone substitute material according to claim 18 **characterised in that** it further comprises one or several network modifier oxides.

20. Bone substitute material according to claim 19 **characterised in that** the network modifier oxide(s) is/are present in a proportion of 0.5 to 35 mole %, preferably in a proportion of 17 to 30 mole %, based on the silica.

21. Bone substitute material according to claim 19 or 20 **characterised in that** the network modifier oxide is Na₂O.

22. Bone substitute material according to claims 18 to 21 **characterised in that** it is obtainable from a bone substitute material according to claims 11 to 17 in which the silica xerogel matrix has been converted partially to completely into the glassy state, the proportion of glass of the matrix being between 0 and 100 vol %, preferably 10 to 80 vol % and particularly preferably between 60 vol % and 80 vol %.

23. Bone substitute material according to claim 21 and 22 **characterised in that** the glass matrix is sodium silicate.

24. Bone substitute material according to claims 18 to 23 **characterised in that** it exhibits a mechanical strength in the region of 30 MPa to 200 MPa, preferably 50 MPa to 120 MPa.

25. Bone substitute material according to claims 18 to 23 **characterised in that** it is a moulded part.

26. Bone substitute material according to claim 25 **characterised in that** the moulded part is a cube, a plate, a hollow cylinder or a wedge.

27. Moulded part of the bone substitute material according to claims 11 to 17 **characterised in that** it comprises on at least one side a layer of a bone substitute material according to claims 18 to 24, holes being contained in this layer with a diameter of 0.5 to 5 mm which exhibit a proportion by volume of 5 to 80%, based on the total volume of the layer and these holes are in turn filled with the granular material according to claims 1 to 10 and/or with the bone substitute material according to claims 11 to 17.

28. Use of a granular material according to claims 1 to 10 for the production of moulded parts.

29. Use according to claim 28 **characterised in that** the moulded part is a cube, a plate, a hollow cylinder or a wedge.

30. Use of a granular material according to claims 1 to 10 for coating implants.

31. Use according to claim 30 **characterised in that** the coating is a plasma spray coating.

32. Use of the granular material according to claims 1 to 10 for the preparation of a medicine or a medicinal product for build-up of osteoporotic bones, for stimulating the bone-build up in the transition area to loosened metal implants or to stimulate healing of parodontal defects.

33. Use according to claim 32 **characterised in that** the granular material is produced by mixing with bone marrow fluid or the patient's own blood and, if necessary, with saline.

34. Medicine or medical product **characterised in that** it comprises granular material according to claims 1 to 10 which is mixed with bone marrow fluid or blood from a patient.

35. Process for the production of a granular material according to claims 1 to 10 **characterised in that** a hydroxyl apatite is precipitated out in aqueous solution, which apatite exhibits, as a result of the ion concentration fixed in the solution, a ratio of Ca/P of 1.50 to 1.67, the pH, the homogeneity of the mixture of the starting products and the temperature being adjusted according to known methods in such a way that crystallites of a size of 10 nm to 2000 nm and granule grains of a size of 1 µm to 1000 µm are formed, the precipitated hydroxyl apatite being homogeneously embedded into a silicon hydrogel without agglomerates forming in the aqueous solution, by supplying silicic acid, preferably orthosilicic acid, to the aqueous solution and adjusting the pH such that it is in the region of 2 to 8, preferably of 5 to 6.5 such that a gel formation takes place, the quantity of silicic acid used being selected in such a way that the proportion of silica is in the region of 2 to 80% by weight, preferably in the region of 4 to 50% by weight, based on the total mass of the granule grains formed, and the hydrogel formed being granulated and subsequently subjected to a drying process for the formation of a xerogel as a result of which the calcium phosphate crystallites are present in a xerogel matrix.

36. Process according to claim 35 **characterised in that** the hydrogel is stored in a closed vessel preferably at room temperature and, if necessary, also at temperatures of approximately 60°C to approximately 80°C, preferably for a period of approximately 24 to 48 hours.

37. Process according to claim 35 and 36 **characterised in that** drying of the hydrogel is carried out preferably at a temperature of 20°C to approximately 150°C, preferably at approximately 120°C.

38. Process for the production of a granular material according to claims 1 to 10 **characterised in that** a hydroxyl apatite is precipitated out in the aqueous solution, which apatite exhibits, as a result of the ion concentration fixed in the solution, a ratio of Ca/P of 1.50 to 1.67, the pH, the homogeneity of the mixture of the starting products and the temperature being adjusted according to known methods in such a way that crystallites of a size of approximately 10 nm to approximately 2000 nm and granule grains of a size of approximately 1 µm to approximately 1000 µm are formed, the precipitated hydroxyl apatite being homogeneously embedded into a silicon hydrogel without agglomerates forming in the aqueous solution, by supplying silicic acid, preferably orthosilicic acid, to the aqueous solution and adjusting the pH such that it is in the region of 2 to 80, preferably of 5 to 6.5 such that a gel formation takes place, the quantity of silicic acid used being selected in such a way that the proportion of silica is in the region of 2 to 80% by weight, preferably in the region of 4 to 50% by weight, based on the total mass of the granule grains formed, spray drying being carried out before gel formation.

39. Process for the production of a granular material according to claims 1 to 10 **characterised in that** a hydroxyl apatite is precipitated out in the aqueous solution, which apatite exhibits, as a result of the ion concentration fixed in the solution, a ratio of Ca/P of 1.50 to 1.67, the pH, the homogeneity of the mixture of the starting products and the temperature being adjusted according to known methods in such a way that crystallites of a size of 10 nm to 2000 nm and granule grains of a size of 1 µm to 1000 µm are formed, the precipitated hydroxyl apatite being homogeneously embedded into a silicon hydrogel without agglomerates forming in the aqueous solution, by supplying silicic acid, preferably orthosilicic acid, to the aqueous solution and adjusting the pH such that it is in the region of 2 to 8, preferably of 5 to 6.5 such that a gel formation takes place, the quantity of silicic acid used being selected in such a way that the proportion of silica is in the region of 2 to 80% by weight, preferably in the region of 4 to 50% by weight, based on the total mass of the granule grains formed, and the hydrogel formed is cooled to temperatures below the freezing point of the solvent and the granular silica/hydroxyl apatite materials is filtered off after thawing.

40. Process according to claims 35, 38 or 39 **characterised in that** calcium nitrate and ammonium hydrophosphate with a ratio of calcium to phosphate (Ca/P ratio) of 1.67 are used to precipitate hydroxyl apatite, the pH being adjusted to approximately 7 to approximately 10.

41. Process according to claims 35, 38 or 39 **characterised in that** calcium nitrate and ammonium hydrophosphate with a ratio of calcium to phosphate (Ca/P ratio) of 1.67 are selected for the precipitation of hydroxyl apatite, a Ca/P ratio of less than 1.67 but more than 1.50 being selected when the granular material is to additionally comprise soluble β-tricalcium phosphate and a Ca/P ration of 1.50 being selected if the granular material is to comprise only soluble β-tricalcium phosphate, the pH being adjusted to 7 to 10.

42. Process according to claims 35 to 41 **characterised in that** the silicic acid is hydrolysed tetraethyl oxysilane (TEOS).

43. Process according to claims 35 to 42 **characterised in that** the pressure is matched to the concentration and the viscosity in such a way that granular materials of 10 µm and less are formed.

44. Process according to claim 43 **characterised in that** the kinematic viscosity amounts to 0.5 to 50 cS.

45. Process for the production of bone substitute material according to claims 11 to 17 **characterised in that**, initially, the process according to claims 35 to 44 is carried out and the granular material obtained is stirred with water to form a slip, approximately 100 to 300 ml of water being preferably added to 100 g of granular material, a pH of preferably between approximately 2 and approximately 8, particularly preferably between approximately 5 and 6.5 being subsequently adjusted, the slip being poured into any desired mould and dried, drying preferably taking place at a temperature between room temperature and 200°C, particularly preferably at a temperature between 80°C and 130°C.

46. Process according to claim 45 **characterised in that** silicic acid, preferably orthosilicic acid, is added to the slip such that, preferably, 1 g to 4 g of silicic acid are present per 100 g of granular material.

47. Process according to claim 46 **characterised in that** the silicic acid is hydrolysed tetraethyl oxysilane (TEOS).

48. Process according to claims 45 to 47 **characterised in that** a further pore structure is produced within the region of magnitude of some hundred µm to the millimetre range by additionally adding to the slip organic powder with a grain size in the pore size desired later which is burnt out after the drying process.

49. Process according to claims 45 to 47 **characterised in that** a further pore structure is produced in the form of continuous pores (channels) in the size region of some 100 µm to the millimetre range by additionally adding to the slip organic fibres of the desired diameter which are burnt out after the drying process.

50. Process according to claim 48 and 49 **characterised in that** the material for the powder or the fibres is wax.

51. Process according to claim 50 **characterised in that** drying of the bone substitute material is carried out at approximately 40°C, the wax is optionally removed from the pores by centrifuging at approximately 100°C, residues of the wax are subsequently burnt out and the carbon formed is removed at approximately 800°C.

52. Process according to claims 45 to 51 **characterised in that** a temperature treatment of approximately 700°C to approximately 900°C is carried out if no network modifiers are present in the granular material used or of approximately 300°C to approximately 500°C if network modifiers are present in the granular material.

53. Process according to claims 45 to 52 **characterised in that** a solution with network modifiers is introduced into the pores of the bone substitute material such that, after drying of the solution, the network modifier oxide is present in a proportion of 0.5 to 35 mol %, preferably in a proportion of 17 to 30 mol %, based on the silica.

54. Process for the production of a bone substitute material according to claims 18 to 26 **characterised in that** a process according to claims 45 to 53 is carried out, network modifier oxide being either contained in the granular material used and/or a network modifier oxide being introduced into the material by the process according to claim 46, the network modifier oxide being present overall in a proportion of 0.5 to 35 mol %, preferably in a proportion of 17 to 30 mol %, based on the silica and the highly porous bone substitute material obtained being subjected to a temperature treatment of approximately 350°C to approximately 800°C in order to convert the xerogel matrix completely or partially into a glass.

55. Process according to claim 54 **characterised in that** two different granular materials according to claims 1 to 10 are used which differ by the proportion of the network modifier, the transition from xerogel to glass taking place only partially as a result during temperature treatment.

56. Process according to claim 55 **characterised in that** a granular material without network modifier (Na₂O) and a granular material with approximately 20 mol % of Na₂O, based on the xerogel, are used.

57. Process according to claim 56 **characterised in that** the temperature treatment is carried out at approximately 520°C, the areas with the Na₂O changing over into the glassy state whereas the areas without Na₂O remaining in the xerogel state.

58. Use of highly porous bone substitute material according to claims 11 to 17 for the preparation of a medicine or a medicinal product for filling small bone defects.

59. Use according to claim 58 **characterised in that** the bone substitute material is present in the form of small pieces.

60. Use according to claim 59 **characterised in that** the pieces are cylinders with an average diameter of 0.4 to 2 mm and a length of 1 to 6 mm.

61. Medicine or medicinal product **characterised in that** it comprises a highly porous bone substitute material according to claims 11 to 17 or a bone substitute material according to claims 18 to 26 whose pores are filled with bone marrow fluid or blood of the patient to be treated.

## Revendications

1. Granulat à base de phosphate de calcium, **caractérisé en ce que** du phosphate de calcium cristallin est dispersé dans une matrice de xérogel de dioxyde de silicium que l'on peut obtenir par fabrication de phosphate de calcium via une réaction de précipitation, dans laquelle on homogénéise la solution avec le phosphate de calcium précipité par agitation, on ajoute une solution d'acide silique fortement concentrée, on fixe le mélange par la formation d'un gel qui survient ensuite et on le transforme par élimination du solvant en une matrice de xérogel, dans lequel les cristallites de phosphate de calcium qui se trouvent dans la matrice de xérogel ont une taille d'environ 10 nm à environ 2000 nm et les grains du granulat ont une taille de 1 µm à 1000 µm, tandis que la fraction de dioxyde de silicium se situe dans la plage de 2 à 80% en poids, de préférence dans la plage de 4 à 50% en poids, par rapport à la masse totale des grains du granulat.

2. Granulat selon la revendication 1, **caractérisé en ce que** les pores du xérogel présentent un diamètre moyen dans la plage de 0,5 nm à 20 nm.

3. Granulat selon la revendication 1 ou 2,
**caractérisé en ce que** les pores des grains de granulat constituent respectivement 10% en volume à 60% en volume par rapport au volume du grain de granulat.

4. Granulat selon les revendications 1 à 3, **caractérisé en ce que** le phosphate de calcium est l'hydroxyapatite.

5. Granulat selon les revendications 1 à 4, **caractérisé en ce que** le granulat comprend par ailleurs du phosphate de calcium soluble.

6. Granulat selon la revendication 5, **caractérisé en ce que** le phosphate de calcium soluble est présent en fraction d'environ 5% en poids à 50% en poids par rapport à la fraction de phosphate de calcium.

7. Granulat selon la revendication 5 ou 6, **caractérisé en ce que** le phosphate de calcium soluble est le phosphate β-tricalcique.

8. Granulat selon les revendications 1 à 7, **caractérisé en ce qu'**il comprend en outre un ou plusieurs oxydes favorisant la formation de réseau.

9. Granulat selon la revendication 8, **caractérisé en ce que** le ou les oxydes favorisant le formation de réseau est ou sont présents en fraction de 0,5 à 35% en mole, de préférence en fraction de 17 à 30% en mole, par rapport au dioxyde de silicium.

10. Granulat selon la revendication 8 ou 9, **caractérisé en ce que** l'oxyde favorisant la formation de réseau est le Na₂O.

11. Matériau de remplacement osseux fortement poreux, **caractérisé en ce qu'**il comprend des grains de granulat du granulat selon les revendications 1 à 10, qui forment une structure tridimensionnelle, qui présente, outre les pores présents dans les grains du granulat, en plus des pores plus ou moins de la taille des grains du granulat.

12. Matériau de remplacement osseux selon la revendication 11, **caractérisé en ce qu'**il présente des macropores interconnectés dans la plage d'environ 100 µm à plusieurs 1000 µm.

13. Matériau de remplacement osseux selon la revendication 11 ou 12, **caractérisé en ce qu'**il présente une porosité totale de 30 à 90% en volume, de préférence de 60 à 80% en volume.

14. Matériau de remplacement osseux selon les revendications 11 à 13, **caractérisé en ce qu'**il présente une résistance à la rupture d'environ 0,1 MPa à 15 MPa, de préférence de 3 MPa à 6 MPa.

15. Matériau de remplacement osseux selon les revendications 11 à 13, **caractérisé en ce qu'**il comprend en outre un ou plusieurs oxydes favorisant la formation de réseau.

16. Matériau de remplacement osseux selon la revendication 15, **caractérisé en ce que** le ou les oxydes favorisant la formation de réseau et ou est ou sont présent(s) en fraction de 0,5 à 35% en mole, de préférence en fraction de 17 à 30% en mole, par rapport au dioxyde de silicium.

17. Matériau de remplacement osseux selon la revendication 15 ou 16, **caractérisé en ce que** l'oxyde favorisant la formation de réseau est le Na₂O.

18. Matériau de remplacement osseux, **caractérisé en ce qu'**il comprend une matrice de verre, dans laquelle est dispersé du phosphate de calcium cristallin, que l'on peut obtenir par fabrication du phosphate de calcium via une réaction de précipitation, dans laquelle on homogénéise la solution avec le phosphate de calcium précipité par agitation, on ajoute une solution d'acide silique fortement concentrée, on fixe le mélange par la formation d'un gel à utiliser par la suite, on le convertit en une matrice de xérogel par élimination du solvant et on convertit ensuite la matrice de xérogel à l'état vitreux avec un agent favorisant ka formation de réseau, dans lequel les cristallites présentent une taille de 10 nm à 2000 nm et la fraction de dioxyde de silicium se situe dans la plage de 2 à 80% en poids, de préférence dans la plage de 4 à 50% en poids, par rapport à la masse totale du matériau de remplacement osseux.

19. Matériau de remplacement osseux selon la revendication 18, **caractérisé en ce qu'**il comprend en outre un ou plusieurs oxydes favorisant la formation de réseau.

20. Matériau de remplacement osseux selon la revendication 19, **caractérisé en ce que** le ou les oxydes favorisant la formation de réseau est ou sont présent(s) en fraction de 0,5 à 35% en mole, de préférence en fraction de 17 à 30% en mole, par rapport au dioxyde de silicium.

21. Matériau de remplacement osseux selon la revendication 19 ou 20, **caractérisé en ce que** l'oxyde favorisant la formation de réseau est le Na₂O.

22. Matériau de remplacement osseux selon les revendications 18 à 21, **caractérisé en ce qu'**on peut l'obtenir à partir de matériau de remplacement osseux selon les revendications 11 à 17, dans lequel la matrice de xérogel de dioxyde de silicium est convertie partiellement à totalement à l'état vitreux, dans lequel la fraction vitreuse de la matrice se situe entre 0 et 100% en volume,' de préférence entre 10 et 80% en volume, tout particulièrement entre 60 et 80% en volume.

23. Matériau de remplacement osseux selon les revendications 21 et 22, **caractérisé en ce que** la matrice vitreuse est le silicate de sodium.

24. Matériau de remplacement osseux selon les revendications 18 à 23, **caractérisé en ce qu'**il présente une résistance mécanique dans la plage de 30 MPa à 200 MPa, de préférence de 50 MPa à 120 MPa.

25. Matériau de remplacement osseux selon les revendications 18 à 23, **caractérisé en ce qu'**il s'agit d'un corps moulé.

26. Matériau de remplacement osseux selon la revendication 25, **caractérisé en ce que** le corps moulé est un parallélépipède, une plaque, un cylindre creux ou un coin.

27. Corps moulé constitué du matériau de remplacement osseux selon les revendications 11 à 17, **caractérisé en ce qu'**il comprend sur au moins une face une couche d'un matériau de remplacement osseux selon les revendications 18 à 24, dans lequel, dans cette couche, il y a des trous d'un diamètre de 0,5 à 5 millimètres, qui représentent une fraction volumique de 5 à 80% par rapport au volume total de la couche et ces trous sont à nouveau remplis par le granulat selon les revendications 1 à 10 et/ou par le matériau de remplacement osseux selon les revendications 11 à 17.

28. Utilisation d'un granulat selon les revendications 1 à 10 pour la fabrication de corps moulés.

29. Utilisation selon la revendication 28,
**caractérisée en ce que** le corps moulé est un parallélépipède, une plaque, un cylindre creux ou un coin.

30. Utilisation d'un granulé selon les revendications 1 à 10 pour le revêtement d'implants.

31. Utilisation selon la revendication 30, **caractérisée en ce que** le revêtement est un revêtement par pulvérisation au plasma.

32. Utilisation d'un granulat selon les revendications 1 à 10 pour la fabrication d'un produit pharmaceutique ou d'un produit médical pour l'élaboration d'os ostéoporeux, pour activer la formation osseuse dans la zone de transition avec des implants métalliques fixés ou pour activer la cicatrisation de défauts parodontaux.

33. Utilisation selon la revendication 32, **caractérisée en ce que** le granulat est fabriqué par mélange avec du liquide de moelle osseuse ou avec le propre sang du patient et éventuellement avec une solution physiologique salée.

34. Produit pharmaceutique ou produit médical, **caractérisé en ce qu'**il comprend un granulat selon les revendications 1 à 10, qui est mélangé à du liquide de moelle osseuse ou avec le sang d'un patient.

35. Procédé de fabrication d'un granulat selon les revendications 1 à 10, **caractérisé en ce que** l'on précipite en solution aqueuse un hydroxyapatite, qui présente du fait des concentrations ioniques fixées dans la solution un rapport Ca/P de 1,50 à 1, 67, dans lequel on règle la valeur du pH, l'homogénéité du mélange des matériaux de départ et la température selon des procédés connus, de sorte qu'il se forme des cristallites d'une taille de 10 nm à 2000 nm et des grains de granulat d'une taille de 1 µm à 1000 µm, dans lequel l'hydroxyapatite précipité est, sans former d'agglomérats dans la solution aqueuse, dispersée de manière homogène dans un hydrogel de silicium dans lequel on achemine une solution aqueuse d'acide silique, de préférence de l'acide orthosilique, et on règle la valeur du pH de sorte qu'elle se situe dans la plage de 2 à 8, de préférence de 5 à 6,5, de sorte qu'il se produise une formation de gel, dans lequel on choisit la quantité utilisée d'acide silique de sorte que la fraction de dioxyde de silicium se situe dans la plage de 2 à 80% en poids, de préférence dans la plage de 4 à 50% en poids, par rapport à la masse totale des grains de granulat qui se forment, et dans lequel on granule l'hydrogel formé et on le soumet ensuite, pour la formation d'un xérogel, à un procédé de séchage, par lequel les cristallites de phosphate de calcium se situent dans une matrice de xérogel.

36. Procédé selon la revendication 35, **caractérisé en ce qu'**on stocke l'hydrogel dans un récipient fermé, de préférence, à température ambiante et éventuellement également à des températures d'environ 60°C à environ 80°C, de préférence, dans un intervalle de temps d'environ 24 à 48 heures.

37. Procédé selon les revendications 35 et 36, **caractérisé en ce que** l'on effectue le séchage de l'hydrogel, de préférence, à une température de 20°C à environ 150°C, mieux encore à environ 120°C.

38. Procédé de fabrication d'un granulat selon les revendications 1 à 10, **caractérisé en ce que** l'on précipite en solution aqueuse un hydroxyapatite, qui présente, du fait des concentrations ioniques fixées dans la solution, un rapport Ca/P de 1,50 à 1,67, dans lequel on règle la valeur du pH, l'homogénéité du mélange des matériaux de départ et la température selon des procédés connus de sorte qu'il se forme des cristallites d'une taille d'environ 10 nm à environ 2000 nm et des grains de granulat d'une taille d'environ 1 µm à environ 1000 µm, dans lequel l'hydroxyapatite précipité, sans former d'agglomérats dans la solution aqueuse, est dispersé de manière homogène dans un hydrogel de silicium dans lequel on achemine la solution aqueuse d'acide silique, de préférence de l'acide orthosilique, et on règle la valeur du pH de sorte qu'elle se situe dans la plage de 2 à 8, mieux encore de 5 à 6,5, de sorte qu'il se produise une formation de gel, dans lequel on choisit la quantité utilisée d'acide silique de sorte que la fraction de dioxyde de silicium se situe dans la plage de 2 à 80% en poids, de préférence dans la plage de 4 à 50% en poids, par rapport à la masse totale des grains de granulat qui se forment et dans lequel on effectue un séchage par pulvérisation avant la formation du gel.

39. Procédé de fabrication d'un granulat selon les revendications 1 à 10, **caractérisé en ce que** l'on précipite en solution aqueuse un hydroxyapatite, qui présente, du fait des concentrations ioniques fixées dans la solution, un rapport Ca/P de 1,50 à 1,67, dans lequel on règle la valeur du pH, l'homogénéité du mélange des matériaux de départ et la température selon des procédés connus de sorte qu'il se forme des cristallites d'une taille de 10 nm à 2000 nm et des grains de granulat d'une taille de 1 µm à 1000 µm, dans lequel l'hydroxylapatite précipité, sans qu'il forme d'agglomérat dans la solution aqueuse, est dispersé de manière homogène dans un hydrogel de silicium, dans lequel on achemine la solution aqueuse d'acide silique, de préférence de l'acide orthosilique, et on régle la valeur du pH de sorte qu'elle se situe dans la plage de 2 à 8, de préférence de 5 à 6,5, de sorte qu'il se forme un gel, dans lequel on choisit la quantité utilisée d'acide silique de sorte que la fraction de dioxyde de silicium se situe dans la plage de 2 à 80% en poids, de préférence dans la plage de 4 à 50% en poids, par rapport à la masse totale des grains de granulat qui se forment, et dans lequel on refroidit l'hydrogel en cours de formation à des températures inférieures au point de congélation du solvant et on sépare par filtration, après décongélation, les granulats de dioxyde de silicium et d'hydroxyapatite qui se forment.

40. Procédé selon les revendications 35, 38 ou 39, **caractérisé en ce que** l'on utilise, pour la précipitation de l'hydroxyapatite, du nitrate de calcium et de l'hydrophosphate d'ammonium avec un rapport calcium sur phosphate (rapport Ca/P) de 1,67, la valeur du pH étant réglée entre environ 7 et environ 10.

41. Procédé selon les revendications 35, 38 ou 39, **caractérisé en ce que** l'on choisit pour la précipitation de l'hydroxyapatite du nitrate de calcium et de l'hydrophosphate d'ammonium avec un rapport calcium sur phosphate (rapport Ca/P) de 1,67, on choisit un rapport Ca/P de moins de 1,67, mais plus grand que 1,50, lorsque le granulat doit comprendre en outre du phosphate β-tricalcique soluble et on choisit un rapport Ca/P de 1,50 lorsque le granulat ne doit contenir que du phosphate β-tricalcique soluble, et dans lequel on règle la valeur du pH entre 7 et 10.

42. Procédé selon les revendications 35 à 41, **caractérisé en ce que** l'acide silique est le tétraéthyloxysilane (TEOS) hydrolysé.

43. Procédé selon les revendications 35 à 42, **caractérisé en ce que** l'on ajuste la pression sur la concentration et la viscosité de sorte qu'il se forme des granulats de 10 µm et moins.

44. Procédé selon la revendication 43, **caractérisé en ce que** la viscosité cinématique est de 0,5 à 50 cst.

45. Procédé de fabrication d'un matériau de remplacement osseux selon les revendications 11 à 17, **caractérisé en ce que** l'on effectue tout d'abord le procédé selon les revendications 35 à 44 et on agite le granulat obtenu avec de l'eau pour obtenir une suspension, dans lequel, de préférence, on ajoute à 100 g de granulat environ 100 à 300 ml d'eau, on règle ensuite une valeur du pH de préférence entre environ 2 et 8, mieux encore entre environ 5 et 6,5, on coule la suspension sous une forme souhaitée et on la sèche, dans lequel le séchage est effectué de préférence à une température entre la température ambiante et 200°C, mieux encore à une température entre 80°C et 130°C.

46. Procédé selon la revendication 45, **caractérisé en ce que** l'on ajoute à la suspension de l'acide silique, de préférence de l'acide orthosilique, de sorte que l'on obtienne de préférence pour 100 g de granulat 1 g à 4 g d'acide silique.

47. Procédé selon la revendication 46, **caractérisé en ce que** l'acide silique est le tétraéthyloxysilane (TEOS) hydrolysé.

48. Procédé selon les revendications 45 à 47, **caractérisé en ce que** l'on produit une autre structure poreuse dans la plage granulométrique de quelques centaines de µm jusque dans la plage du mm en ajoutant à la suspension en outre une poudre organique avec une granulométrie dans la taille de pores souhaitée par la suite, qui a été calcinée après le processus de séchage.

49. Procédé selon les revendications 45 à 47, **caractérisé en ce que** l'on produit une autre structure poreuse sous la forme de pores continus (canaux) dans la plage granulométrique de quelques centaines de µm jusque dans la plage des mm en ajoutant en plus à la suspension des fibres organiques de diamètre souhaité, qui sont calcinées après le processus de séchage.

50. Procédé selon les revendications 48 et 49, **caractérisé en ce que** le matériau pour la poudre ou les fibres est la cire.

51. Procédé selon la revendication 50, **caractérisé en ce qu'**on effectue le séchage du matériau de remplacement osseux à environ 40°C, on élimine des pores la cire facultativement par centrifugation à environ 100°C, on calcine ensuite le reste de la cire et on élimine à environ 800°C le carbone formé.

52. Procédé selon les revendications 45 à 51, **caractérisé en ce qu'**on effectue un traitement à la chaleur d'environ 700°C à environ 900°C lorsqu'aucun agent favorisant la formation de réseau n'est présent dans le granulat utilisé, ou d'environ 300°C à environ 500°C, lorsque des agents favorisant la formation de réseau sont présents dans le granulat.

53. Procédé selon les revendications 45 à 52, **caractérisé en ce qu'**une solution avec des agents favorisant la formation de réseau est acheminée dans les pores du matériau de remplacement osseux de sorte qu'après séchage de la solution, l'oxyde favorisant la formation de réseau soit présent en fraction de 0,5 à 35% en mole, de préférence en fraction de 17 à 30% en mole, par rapport au dioxyde de silicium.

54. Procédé de fabrication d'un matériau de remplacement osseux selon les revendications 18 à 26, **caractérisé en ce que** l'on effectue un procédé selon les revendications 45 à 53, dans lequel de l'oxyde favorisant la formation de réseau est contenu dans le granulat utilisé et/ou on introduit par le procédé de la revendication 46 un oxyde favorisant la formation de réseau dans les matériaux, dans lequel l'oxyde favorisant la formation de réseau se présente au total en fraction de 0,5 à 35% en mole, de préférence en fraction de 17 à 30% en mole, par rapport au dioxyde de silicium et on soumet le matériau de remplacement osseux fortement poreux obtenu à un traitement à la chaleur d'environ 350°C à environ 800°C pour convertir la matrice de xérogel totalement ou partiellement en un verre.

55. Procédé selon la revendication 54, **caractérisé en ce que** l'on utilise deux granulats différents selon les revendications 1 à 10, qui se distinguent par la fraction d'agent favorisant la formation de réseau, par lequel, lors du traitement à la chaleur, la transition entre xérogel et verre ne se fait plus que partiellement.

56. Procédé selon la revendication 55, **caractérisé en ce que** l'on utilise un granulat sans agent favorisant la formation de réseau (Na₂O) et un granulat avec environ 20% en mole de Na₂O par rapport au xérogel.

57. Procédé selon la revendication 56, **caractérisé en ce que** l'on effectue le traitement à la chaleur d'environ 520°C, dans lequel les zones avec le Na₂O fusionnent à l'état vitreux, tandis que les zones sans Na₂O restent à l'état de xérogel.

58. Utilisation de matériaux de remplacement osseux fortement poreux selon les revendications 11 à 17 pour la fabrication d'un produit pharmaceutique ou d'un produit médical pour combler de petites imperfections osseuses.

59. Utilisation selon la revendication 58, **caractérisée en ce que** le matériau de remplacement osseux se présente sous la forme de petites pièces.

60. Utilisation selon la revendication 59, **caractérisée en ce que** les petites pièces sont des cylindres avec un diamètre moyen de 0, 4 à 2 mm et une longueur de 1 à 6 mm.

61. Produit pharmaceutique ou produit médical, **caractérisé en ce qu'**il comprend un matériau de remplacement osseux fortement poreux selon les revendications 11 à 17 ou un matériau de remplacement osseux selon les revendications 18 à 26, dont les pores sont remplis par le liquide de moelle osseuse ou par le sang du patient à traiter.
